# EUROPEAN PATENT APPLICATION

(11) **EP 2 042 188 A1**
(43) Date of publication of application: **01.04.2009**
(21) Application number: 07743451.2
(22) Date of filing: 16.05.2007
(51) Int. Cl.: A61K 38/21, A61K 45/00, A61P 1/16, A61P 31/12, A61P 35/00

(54) **PHARMACEUTICAL COMPOSITION FOR TREATING OR PREVENTING HCV INFECTION**

(30) Priority: 16.05.2006 JP 2006136992
(71) Applicant: Tokyo Metropolitan Organization for Medical Research, Shinjuku-ku, Tokyo 163-8001 (JP); CHUGAI SEIYAKU KABUSHIKI KAISHA, Tokyo, 115-8543 (JP)
(72) Inventor: KOHARA, Michinori, Tokyo 114-0014 (JP); UMEHARA, Takuya, Tokyo 114-0014 (JP); SUDO, Masayuki, Kanagawa 247-8530 (JP)
(74) Representative: Vossius & Partner
(86) International application number: PCT/JP2007/060016
(87) International publication number: WO 2007/132882

(57) **Abstract**

The present inventors examined the anti-HCV effect of SPT inhibitors and/or interferon using HCV subgenomic replicon cell FLR3-1 and chimeric mice infected with HCV. The results demonstrate that myriocin and the compound of formula (III), which are SPT inhibitors, can serve as novel therapeutic agents for HCV infection, and that the combined use of these SPT inhibitors and interferon provides a stronger effect of inhibiting HCV replication.

SEQUENCE LISTING

<110> TOKYO METROPOLITAN ORGANIZATION FOR MEDICAL RESEARCH
CHUGAI SEIYAKU KABUSHIKI KAISHA

<120> PHARMACEUTICAL COMPOSITION FOR TREATING OR PREVENTING HCV INFECTION

<130> C1-A0608P

<150> JP 2006-136992
<151> 2006-05-16

<160> 10

<170> PatentIn version 3.3

<210> 1
<211> 21
<212> RNA
<213> Artificial

<220>
<223> An artificially synthesized siRNA sequence

<220>
<221> misc_feature
<222> (20)..(21)
<223> n indicates dT

<400> 1
caucguuuca ggcccuccan n 21

<210> 2
<211> 21
<212> RNA

<213>  Artificial

<220>
<223>  An artificially synthesized siRNA sequence

<220>
<221>  misc_feature
<222>  (20)..(21)
<223>  n indicates dT

<400>  2
ugacauggcu gaccucgagn n                                                21

<210>  3
<211>  2742
<212>  DNA
<213>  Homo sapiens

<220>
<221>  CDS
<222>  (9)..(1550)

<400>  3
   ggctaact atg gcg acc gcc acg gag cag tgg gtt ctg gtg gag atg gta       50
   cag gcg ctt tac gag gct cct gct tac cat ctt att ttg gaa ggg att        98
   ctg atc ctc tgg ata atc aga ctt ctt ttc tct aag act tac aaa tta       146
   caa gaa cga tct gat ctt aca gtc aag gaa aaa gaa gaa ctg att gaa 194
   gag tgg caa cca gaa cct ctt gtt cct cct gtc cca aaa gac cat cct 242
   gct ctc aac tac aac atc gtt tca ggc cct cca agc cac aaa act gtg 290
   gtg aat gga aaa gaa tgt ata aac ttc gcc tca ttt aat ttt ctt gga 338
   ttg ttg gat aac cct agg gtt aag gca gca gct tta gca tct cta aag 386
   aag tat ggc gtg ggg act tgt gga ccc aga gga ttt tat ggc aca ttt 434
   gat gtt cat ttg gat ttg gaa gac cgc ctg gca aaa ttt atg aag aca 482
   gaa gaa gcc att ata tac tca tat gga ttt gcc acc ata gcc agt gct 530
   att cct gct tac tct aaa aga ggg gac att gtt ttt gta gat aga gct 578
   gcc tgc ttt gct att cag aaa gga tta cag gca tcc cgt agt gac att 626
   aag tta ttt aag cat aat gac atg gct gac ctc gag cga cta cta aaa 674
   gaa caa gag atc gaa gat caa aag aat cct cgc aag gct cgt gta act 722
   cgg cgt ttc att gta gta gaa gga ttg tat atg aat act gga act att 770
   tgt cct ctt cca gaa ttg gtt aag tta aaa tac aaa tac aaa gca aga 818
   atc ttc ctg gag gaa agc ctt tca ttt gga gtc cta gga gag cat ggc 866
   cga gga gtc act gaa cac tat gga atc aat att gat gat att gat ctt 914
   atc agt gcc aac atg gag aat gca ctt gct tct att gga ggt ttc tgc 962
   tgt ggc agg tct ttt gta att gac cat cag cga ctt tcc ggc cag gga 1010
   tac tgc ttt tca gct tcg tta cct ccc ctg tta gct gct gca gca att     1058
   gag gcc ctc aac atc atg gaa gag aat cca ggt att ttt gca gtg ttg     1106
   aag gaa aag tgc gga caa att cat aaa gct tta caa ggc att tct gga     1154
   tta aaa gtg gtg ggg gag tcc ctt tct cca gcc ttt cac cta caa ctg     1202
   gaa gag agc act ggg tct cgc gag caa gat gtc aga ctg ctt cag gaa     1250
   att gta gat caa tgc atg aac aga agt att gca tta act cgg gcg cgc     1298
   tac ttg gag aaa gaa gag aag tgt ctc cct cct ccc aga gga aga act     1346
   gga gag agc tgc gtc cac cat caa gga ggt agc cca ggc cgt cct gct     1394
   cta ggc aga gtc ccg gga cca tgg cct cct gcc aca caa cac gca gag     1442
   agg act caa gac tcc cgc tgg cca tgg agt ggc ctg aaa gag agc aag 1490
   aac atg tgg atc ttt gat agg att gtt acc aaa tgg tgt cag tat gga 1538
   cca att gtg tga ccatgagaag gatgcttatt ttttttaaaa agaaaacaca         1590
   tctaaaagcc caggaactga tttttttaag aggaaaacta atgacagtgt ataactgatg 1650
   tttaaattgt gcatttagta ctatttaaat gttttcttat actagtattt tatattcttt 1710
   tgttgtcgtt taaaactgga gcttcagtgt ctcttccctc cctctaatag taatggttca 1770
   gtaagcactc cttaactcct tagtatttca tagaaaaatg actgcaacat taaagctaag 1830
   aggaacactt caacatatgt ggtacaaatt tatattgaag atctaaataa accacgtatt 1890
   ttccagtctt cgttgtgtga agctaaatgg tggctaaaag gaacactttt tgtgtgatta 1950
   ttataaactt tgcattgtat ttgaatctta gaacttttgt acacactaaa tattgatgtc 2010
   acaccatttc taatctgagc atccttagcc agagaatatt cattatactt cctaagtgag 2070
   caataattta aatcagaagc tattttattt taatgtaatt aacctttctt tacatttctt 2130
   atgtgttcac ctctaatctg ttttaggaag agagttggtt attatgttga tcccataata 2190
   taaatcatat cctttatatt ttagaatatc tcaaatgtat tccttttttg tatggtgggt 2250
   ttgcctaggg acgtgtaact acaggctttt actaagccaa ggaaaaagag aatttttctt 2310
   ttcatcttac aaattccaga tatctacaaa agatgtgaaa gcactaaaaa taccattttt 2370
   aagcagtact ttacctgttt tttctttagc aaaccaggtt atgtggtgta aaggtttgtt 2430
   atacgtgcca caatatagca tataaatatt atgccatcat tccttctctt gttaaaggta 2490
   gaagaataaa attgtgattt ttataacctg tgcttattac tcaaatggtc ttcaacatct 2550
   ttttaaacaa cacatacttt ttgaatgttc agtttctatt ttgcttgagg tattttgtac 2610
   atatgtgcct tgtgattgct gctgctttaa aggataaagt actctttggg ggatgagtct 2670
   ggtttgtttt gttttatttt ttaatgaaat aaacctatat tcctgattat tagtccaaaa 2730
   aaaaaaaaaa aa 2742

<210> 4
<211> 513
<212> PRT
<213> Homo sapiens

<400> 4

<210> 5
<211> 7250
<212> DNA
<213> Homo sapiens

<220>
<221> CDS
<222> (189)..(1877)

<400> 5
ccttggccga gaccggtcct ctgcggagag ggccccgccc tctgtgaagg cccgcccggg 60
aattggcggc ggcgctgcag ccatttccgg tttcggggag gtgggtgggg tgcggagcgg 120
gacttggagc agccgccgcc gctgccaccg cctacagagc ctgccttgcg cctggtgctg 180
ccaggaag atg cgg ccg gag ccc gga ggc tgc tgc tgc cgc cgc acg gtg 230
cgg gcg aat ggc tgc gtg gcg aac ggg gaa gta cgg aac ggg tac gtg 278
agg agc agc gct gca gcc gca gcc gca gcc gcc gcc ggc cag atc cat 326
cat gtt aca caa aat gga gga cta tat aaa aga ccg ttt aat gaa gct 374
ttt gaa gaa aca cca atg ctg gtt gct gtg ctc acg tat gtg ggg tat 422
ggc gta ctc acc ctc ttt gga tat ctt cga gat ttc ttg agg tat tgg 470
aga att gaa aag tgt cac cat gca aca gaa aga gaa gaa caa aag gac 518
ttt gtg tca ttg tat caa gat ttt gaa aac ttt tat aca agg aat ctg 566
tac atg agg ata aga gac aac tgg aat cgg cca atc tgt agt gtg cct 614
gga gcc agg gtg gac atc atg gag aga cag tct cat gat tat aac tgg 662
tcc ttc aag tat aca ggg aat ata ata aag ggt gtt ata aac atg ggt 710
tcc tac aac tat ctt gga ttt gca cgg aat act gga tca tgt caa gaa 758
gca gcc gcc aaa gtc ctt gag gag tat gga gct gga gtg tgc agt act 806
cgg cag gaa att gga aac ctg gac aag cat gaa gaa cta gag gag ctt 854
gta gca agg ttc tta gga gta gaa gct gct atg gcg tat ggc atg gga 902
ttt gca acg aat tca atg aac att cct gct ctt gtt ggc aaa ggt tgc 950
ctg att ctg agt gat gaa ctg aat cat gca tca ctg gtt ctg gga gcc 998
aga ctg tca gga gca acc att aga atc ttc aaa cac aac aat atg caa 1046
agc cta gag aag cta ttg aaa gat gcc att gtt tat ggt cag cct cgg 1094
aca cga agg ccc tgg aag aaa att ctc atc ctt gtg gaa gga ata tat 1142
agc atg gag gga tct att gtt cgt ctt cct gaa gtg att gcc ctc aag     1190
aag aaa tac aag gca tac ttg tat ctg gat gag gct cac agc att ggc     1238
gcc ctg ggc ccc aca ggc cgg ggt gtg gtg gag tac ttt ggc ctg gat     1286
ccc gag gat gtg gat gtt atg atg gga acg ttc aca aag agt ttt ggt     1334
gct tct gga gga tat att gga ggc aag aag gag ctg ata gac tac ctg     1382
cga aca cat tct cat agt gca gtg tat gcc acg tca ttg tca cct cct     1430
gta gtg gag cag atc atc acc tcc atg aag tgc atc atg ggg cag gat     1478
ggc acc agc ctt ggt aaa gag tgt gta caa cag tta gct gaa aac acc     1526
agg tat ttc agg aga cgc ctg aaa gag atg ggc ttc atc atc tat gga     1574
aat gaa gac tct cca gta gtg cct ttg atg ctc tac atg cct gcc aaa 1622
att ggc gcc ttt gga cgg gag atg ctg aag cgg aac atc ggt gtc gtt 1670
gtg gtt gga ttt cct gcc acc cca att att gag tcc aga gcc agg ttt 1718
tgc ctg tca gca gct cat acc aaa gaa ata ctt gat act gct tta aag 1766
gag ata gat gaa gtt ggg gac cta ttg cag ctg aag tat tcc cgt cat 1814
cgg ttg gta cct cta ctg gac agg ccc ttt gac gag acg acg tat gaa 1862
gaa aca gaa gac tga gcctttttgg tgctccctca gaggaactct ccctcaccca 1917
ggacagcctg tggcctttgt gagccagttc caggaaccac acttctgtgg ccatctcacg 1977
tgaaagacat tgcctcagct actgaaggtg gccacctcca ctctaaatga cattttgtaa 2037
atagtaaaaa actgcttcta atccttcctt tgctaaatct cacctttaaa aacgaaggtg 2097
actcactttg ctttttcagt ccattaaaaa aacattttat tttgcaacca ttctacttgt 2157
gaaatcacgc tgaccctagc ctgtctctgg ctaaccacac aggccattcc cctctcccag 2217
caccttgcag acttgggccc atcaagagct actgctggcc ctggctccgc agcctggata 2277
cttacctggc cctcctccct agggagcaag tgccttccac ttacttccca tccaggtctc 2337
agaggtctca aggccaacct tggaatcctt atttaaccat tcaagtaatc aacggaagtt 2397
ttcacccttt aatcttaagt ttagcctttt aagaaaaaca gtaagcgatg actgctgaaa 2457
ggctcattgt gtaatctccc aagggtttgg tcttattcca ttttcttctg gtcaccagat 2517
gatttcttcc tttaccatca aatacttctt cataatggtc acagtctgag gatgtgcgca 2577
aattctggtt cttcccaagc tctaaccgta acacgtccca cccccttttt aaagcactta 2637
ctgttttcag agcacccata tcccaccctg gtgagaaggc cactctcaca tctgagtgtt 2697
gggtacaaag ctgctccgta gagtgatgtg cactcctggt gggtgagggg caggggcagt 2757
ggcagtgtgc aaagaattga ttactccttg cagagcctgt ggcttgcatt tcctactgct 2817
ttctacgttt gaaaattatg acagtctctg gctaggtctg ggtccagatt aggatttaaa 2877
ctgataaagg aaactgttgg taaatcctct gctcagaaag catttatcat gttcctattt 2937
aaggattagg tttattaatt taggcctctt agaagctaac ccacttaaat attactcttc 2997
tgaatgctag ttctctttta ttcttgatgt cctaagtcaa ttgaatctgg catctggggc 3057
tagggtctgc ctgtctacat attttttatt tttttctgag aaattctgaa cacatagatc 3117
tctttcctaa actgacattt tctattttga ctgttttcat actataacca ggtaaaggga 3177
cttctttcag agagctttat actgcctgac caaagaacaa atctgaaaat caccatttta 3237
aagttatttt ttcagttgaa ccaaagttta agtgaagagg acttttggca tattataccc 3297
aggatcagtt tgtctttttg tatccatcaa gtattacagg agaaggattg ggaacagaat 3357
ggaaaaacag tgtatgaaag tcatgttaca ggccgagtgc ggtggctcac acctgtaatc 3417
ctagcacttt gggaggctga ggcaggtggc tcacttgagg tcaggaattc aagaccagcc 3477
tggccaacat ggtgaaaccc cgtctctact aaaaagacaa aaaattagct gggcgtggtg 3537
gcgggcacct ataatcccac ctacttggta ggctgaggca ggagaatcgc ttgaacccag 3597
gaggcggagg ttgcagtgag acgagattgt gccactgcac tctagcctgg gtgacagagc 3657
aaaactgtgt ctcaaaaaaa aaagtcatgt tacacattta agtttttgaa attgctcctt 3717
ttatcggtaa agattctcaa tccaaattct cctgggtgtg ttgtcatcag ctgtgatatg 3777
tttgtgcaca ttacgtatag cagaggatgt aagcaatatt attgtttgtg aagttttgtt 3837
tttaatgtct tgagtatgag ttatgtttag tcactgtcag catctgagaa ctttaataag 3897
cccttgagat attccaaagt tttattttac ttttttaaag aacagaaaaa gatgaatgaa 3957
agaaccaagg agagatgcag agactatatt tagcatgtat aggttaaagt aagaaggagg 4017
ttgtggtaac taaataggag tcctataaaa tcaaatacat tgtcaacctt ttctgcacat 4077
ctagtttcct accatagaat cccactggaa taccacatag cttttgcact gcagttacta 4137
tttactaatg taaacgtagg gtttgtaaaa gtcacaaact tataagcaat gaacttacct 4197
gctagtcttt ttattttggc ttgcatgaag tcactgcaaa ttcaaatgtc agtaccggca 4257
tttaaaatat atctatatca ctttgttggt acaaagttat ttcaagataa gtgtaatttt 4317
gttacaagtt tattttgaag agacaaatct cctgtgatct atgcaggacc tctgtacttt 4377
ctaaagaaca aaatgttatg tagacattat acatggttgg ttgtctcttc ttgaaactgt 4437
aatgtaaatc tagggtccag tcatatccta ggtatcatca tttatccaag tacttggagg 4497
aatacaagta tatataaata cagtcattga gaataagtcg atttgaggca tacaagagta 4557
gtttcttaca cagtttaaca cggcctgatt caagactctg ataggattca aacagatacc 4617
ggttaaccat gactaccaaa actgatcatc tgagtcgatt gatagaggtg tgactagtcc 4677
ttagcacttt ttctcattcc tctttttatt cagcattgct gttacctatt tcaggtttat 4737
aagacctctt tcagcagatc acatcagaag ccaggaaatg catagctagg agatgtcaaa 4797
agcccatatg aggagtggac caagcagcag tggcggtttc tcctcgcatc tttttttttt 4857
taagctttaa cttagcaggg gcatggactt tatagcactt tttcaacttt ttgctttgct 4917
ttggataaga aatccttacc tttaaaaaaa gcttctagtc tccataaccc ccaaagtact 4977
gcttatttgt ttgaagaatc cagccatcgt agtgctttag tcactatcgt aaacattcat 5037
gatagggcaa ggattttaaa acaggattct tgcttctgta gtcatcaagg tgaacagaag 5097
catcctacac aaccactaag ggctctatgt ttgtgtcatg cctcttcaaa caccaaggag 5157
ttgaacatgc ttccagtgat ttgtctccgt aatgccttct tcctttattt ggcctttctt 5217
tctttctgta ccttcaagtt cttgattttt aaaattccaa ctctagagaa aaccaatata 5277
tggtggtgct gggctttgaa gatagcatat cagacgcctt ggttctgttt gtacacttag 5337
ccttacattt caggaggagg cttttcatta ggggcttaag ctagctcctt tggcttttaa 5397
aaaaaatttt ttttcaaatt tcttcattac ctaagggagc ctgcatctaa atttctcaac 5457
tagttcagcc tagctgaatt ttctagtgtg taatacactt tgcttccttc ttattggtga 5517
aaaccagggg gatgagtggc ttccatggag agatttcctg atttctcagg gaggaaaaaa 5577
gtgatgacat ttaccactac ttttatgttt ttcccctttt tccaaattga taaggatttc 5637
tggttcctag tgatccggga ttgggcaaca gtgcagaact gccagtcatg ccgtaggccg 5697
tgaagaaaga atgtgagtaa ctgttgtttt gcaaggattt gtagggttat gggcagttgt 5757
tgtttgaagc attgctatga cctaattccc aaggtatctt tcctctcttg gtgttctagg 5817
taagccaatg agctttaatc tctacttgct ataaccgtgt gcttagaaaa agaggtgaga 5877
gtagtggttt tccttcaaac tgtccacatt catgaagatt atgaattgtt aggacagcca 5937
gggcaagata gaccctgtct ctacaaaaat ttttttctaa attaaccggg catggtggtg 5997
cctgcctgta gtcccacctg tgtgggagaa tcacttgagc ctgggaggtc aaggctgcag 6057
tgagccatga ttgcacccct gcactccagc ctgggtgaca gagtgagacc ctggctcaat 6117
aagaggggga aaaaaaattg ttaggagctg ggtgcggatg cagcctgcaa tcccagctac 6177
ttgagaggct gaggccggag gattgcttaa acccaagaat ttgagcgtag cctgggcaac 6237
acagcaagac cccatctaag aaaaaaatgt tttttaaatc agcttagccc aaaggggttg 6297
tgaatgggga ggtataaaaa gcaaagatta ttttttggct actaagccaa gaacttacag 6357
ggattttttt tttcagtccc agaacctaca gataccctgc tacttgcttc acgtggatgc 6417
tcagtgccca gcagccatct taatacatta aaccagttta aaaaatacct tccatgtgga 6477
gaaaaacatg tctttttctc gcctcaactt tatccacatg aaatgtgtgc ccatggctgg 6537
gcgcagtggc tcacctgtaa tcccaacact ttgggaggct gaagcaggca gattgcttga 6597
ggccaggagt tcgagaacag tctggccaac atggcgaaac ctcatctcta ctaaaattac 6657
aaaaattagc cgggcatggt ggcacatgcc tgtaatccca gctacgtcag gaggctgagg 6717
cacaggaatt gcttgaaccc aagaggcaga ggatgcaatg agccaagatc acaccactgc 6777
actccagcct tggcgacaga gggagactct gtctcaaaaa aaaaaaaaaa aggtgtgccc 6837
aggcccctag ccattgccat gtgcccagcc agagagccaa attagagggc tggcttccct 6897
atcacacaga ataaatgcta gtgctagcca atgatccctt tgcttttaat gtatagaaaa 6957
tactgttgtt ccttttgtca tttccagtga catctgtttt ctaagcagct cttttctagg 7017
gaggaaacca aaggggctag gttaagaccc taatagaaat gttttttcta atctctggtg 7077
agtctggaag tgtcacattc acagtccacc cttgggagtg gcttggtgga gctggggaca 7137
aggttttgtt tactacatag tgcacatgat aaatggcctt aaactgtgat tctttctggt 7197
aggataagtt ataataaact gaccctaaag aatgcaaaaa aaaaaaaaaa aaa 7250

<210> 6
<211> 562
<212> PRT
<213> Homo sapiens

<400> 6

<210> 7
<211> 189

<212>  PRT
<213>  Homo sapiens

<400>  7

Met Ala Ser Pro Phe Ala Leu Leu Met Val Leu Val Val Leu Ser Cys
1               5                   10                  15

Lys Ser Ser Cys Ser Leu Gly Cys Asp Leu Pro Glu Thr His Ser Leu
            20                  25                  30

Asp Asn Arg Arg Thr Leu Met Leu Leu Ala Gln Met Ser Arg Ile Ser
        35                  40                  45

Pro Ser Ser Cys Leu Met Asp Arg His Asp Phe Gly Phe Pro Gln Glu
    50                  55                  60

Glu Phe Asp Gly Asn Gln Phe Gln Lys Ala Pro Ala Ile Ser Val Leu
65                  70                  75                  80

His Glu Leu Ile Gln Gln Ile Phe Asn Leu Phe Thr Thr Lys Asp Ser
                85                  90                  95

Ser Ala Ala Trp Asp Glu Asp Leu Leu Asp Lys Phe Cys Thr Glu Leu
            100                 105                 110

Tyr Gln Gln Leu Asn Asp Leu Glu Ala Cys Val Met Gln Glu Glu Arg
        115                 120                 125

Val Gly Glu Thr Pro Leu Met Asn Ala Asp Ser Ile Leu Ala Val Lys
    130                 135                 140

Lys Tyr Phe Arg Arg Ile Thr Leu Tyr Leu Thr Glu Lys Lys Tyr Ser
145                 150                 155                 160

Pro Cys Ala Trp Glu Val Val Arg Ala Glu Ile Met Arg Ser Leu Ser
                165                 170                 175

Leu Ser Thr Asn Leu Gln Glu Arg Leu Arg Arg Lys Glu
            180                 185

<210> 8
<211> 570
<212> DNA
<213> Homo sapiens

<400> 8
atggcctcgc cctttgcttt actgatggtc ctggtggtgc tcagctgcaa gtcaagctgc 60

tctctgggct gtgatctccc tgagacccac agcctggata acaggaggac cttgatgctc 120

ctggcacaaa tgagcagaat ctctccttcc tcctgtctga tggacagaca tgactttgga 180

tttccccagg aggagtttga tggcaaccag ttccagaagg ctccagccat ctctgtcctc 240

catgagctga tccagcagat cttcaacctc tttaccacaa aagattcatc tgctgcttgg 300

gatgaggacc tcctagacaa attctgcacc gaactctacc agcagctgaa tgacttggaa 360

gcctgtgtga tgcaggagga gagggtggga gaaactcccc tgatgaatgc ggactccatc 420

ttggctgtga agaaatactt ccgaagaatc actctctatc tgacagagaa gaaatacagc 480

ccttgtgcct gggaggttgt cagagcagaa atcatgagat ccctctcttt atcaacaaac 540

ttgcaagaaa gattaaggag gaaggaataa 570

<210> 9
<211> 166
<212> PRT
<213> Homo sapiens

<400> 9

Met Lys Tyr Thr Ser Tyr Ile Leu Ala Phe Gln Leu Cys Ile Val Leu
1               5                   10                  15

Gly Ser Leu Gly Cys Tyr Cys Gln Asp Pro Tyr Val Lys Glu Ala Glu
            20                  25                  30

Asn Leu Lys Lys Tyr Phe Asn Ala Gly His Ser Asp Val Ala Asp Asn
        35                  40                  45

Gly Thr Leu Phe Leu Gly Ile Leu Lys Asn Trp Lys Glu Glu Ser Asp
    50                  55                  60

Arg Lys Ile Met Gln Ser Gln Ile Val Ser Phe Tyr Phe Lys Leu Phe
65                  70                  75                  80

Lys Asn Phe Lys Asp Asp Gln Ser Ile Gln Lys Ser Val Glu Thr Ile

                85                  90                  95

Lys Glu Asp Met Asn Val Lys Phe Phe Asn Ser Asn Lys Lys Lys Arg
            100                 105                 110

Asp Asp Phe Glu Lys Leu Thr Asn Tyr Ser Val Thr Asp Leu Asn Val
        115                 120                 125

Gln Arg Lys Ala Ile His Glu Leu Ile Gln Val Met Ala Glu Leu Ser
    130                 135                 140

Pro Ala Ala Lys Thr Gly Lys Arg Lys Arg Ser Gln Met Leu Phe Arg
145                 150                 155                 160

Gly Arg Arg Ala Ser Gln
                165

<210> 10
<211> 501
<212> DNA
<213> Homo sapiens

<400> 10
atgaaatata caagttatat cttggctttt cagctctgca tcgttttggg ttctcttggc 60

tgttactgcc aggacccata tgtaaaagaa gcagaaaacc ttaagaaata ttttaatgca 120

ggtcattcag atgtagcgga taatggaact cttttcttag gcattttgaa gaattggaaa 180

gaggagagtg acagaaaaat aatgcagagc caaattgtct ccttttactt caaacttttt 240

aaaaacttta aagatgacca gagcatccaa aagagtgtgg agaccatcaa ggaagacatg 300

aatgtcaagt ttttcaatag caacaaaaag aaacgagatg acttcgaaaa gctgactaat 360

tattcggtaa ctgacttgaa tgtccaacgc aaagcaatac atgaactcat ccaagtgatg 420

gctgaactgt cgccagcagc taaaacaggg aagcgaaaaa ggagtcagat gctgtttcga 480

ggtcgaagag catcccagta a 501

## Description

### Technical Field

The present invention relates to pharmaceutical compositions for treating or preventing HCV infection, which comprise myriocin and an interferon as active ingredients, and uses thereof.

### Background Art

Hepatitis C (HCV) infection generally causes chronic hepatitis, and often leads to cirrhosis or hepatocellular carcinoma. At present, HCV carriers account for about 3% of the population (about 170 million) in the world. Since HCV escapes host immune systems by unknown causes, it often establishes persistent infection even in adults having well-developed immune systems, which progresses to chronic hepatitis, cirrhosis, and hepatic cancer. It is known that even patients who have undergone surgical removal often have recurrence of the cancer due to continuous inflammation in noncancerous tissues.

The most effective method for treating HCV infection that is currently known is the combined use of a pegylated interferon (PEG-IFN) and ribavirin (Non-Patent Documents 1 and 2). However, interferon therapy is effective for only about one third of the total patients, and in particular the efficacy of interferon against HCV genotype 1b is very low. Thus, there is a need to develop anti-HCV agents that can be used instead of or in combination with interferons. In particular, besides symptomatic therapy which uses anti-inflammatory agents to suppress inflammation, there is a strong demand for agents to reduce or eradicate HCV in the affected liver.

HCV is a single-stranded RNA virus belonging to Flaviviridae. Its RNA genome produces at least ten viral proteins, including structural and nonstructural (NS) proteins. The structural proteins are involved in the formation of HCV particles. The nonstructural proteins play an important role in HCV genome replication (Non-Patent Document 3). It is generally accepted that the NS protein complex becomes associated with lipid rafts on the membrane of Golgi apparatus and endoplasmic reticulum, whereby HCV infection occurs (Non-Patent Documents 4 and 5). Thus, disruption of lipid raft aggregates may result in suppression of HCV replication.

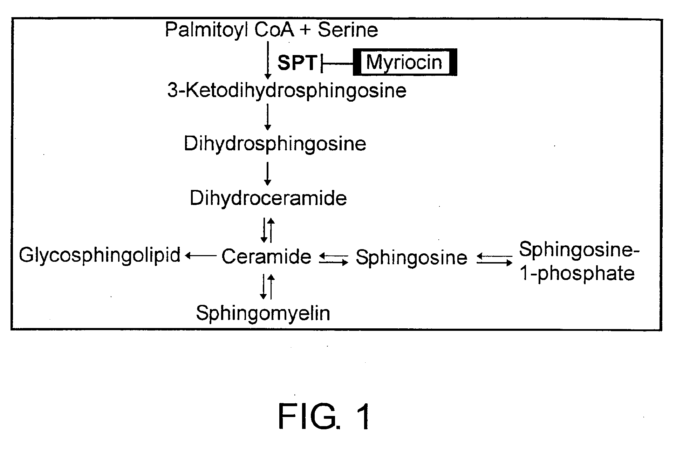

Myriocin (ISP-1) is a specific inhibitor of serine palmitoyltransferase (SPT) which is the first step enzyme in the sphingolipid biosynthetic pathway (Fig. 1; Non-Patent Documents 6 and 7). Because of structural similarity to sphingosine, myriocin inhibits the SPT activity and thereby reduces intracellular sphingomyelin and its intermediates, namely dihydrosphingosine, sphingosine, ceramide, and sphingosine-1-phosphate (Fig. 1). Since sphingomyelin is an essential component of lipid raft aggregates, the inhibition of SPT by myriocin may finally result in the disruption of lipid rafts (Non-Patent Document 8).

Recently, Sakamoto *et al.* have isolated NA255, a compound that suppresses the replication of HCV subgenomic replicons (Non-Patent Document 9 and Patent Document 1). It is known that NA255 is structurally similar to myriocin and inhibits the enzymatic activity of SPT, thereby resulting in the suppression of HCV replication without affecting the enzymatic activity of HCV NS3 (protease and helicase) or NS5B (RNA-dependent RNA polymerase). Thus, the replication of HCV subgenomic replicons was suppressed by NA255 in response to the reduction in the levels of sphingolipids, ceramide, and sphingomyelin. These findings suggest that NA255 destroys lipid raft aggregates associated with HCV NS proteins.

The results described above have suggested that myriocin produces an anti-HCV effect. However, it has not been elucidated what effect the combination of myriocin and interferon has on the inhibition of HCV replication.

Prior art documents related to the present invention are shown below:
Patent Document 1: WO2006/016657
Non-Patent Document 1: Glue, P. et al., Hepatology, 32, 647-653 (2000)
Non-Patent Document 2: Reddy, K.R. et al., Hepatology, 33, 433-438 (2001)
Non-Patent Document 3: Rosenberg, S., J Mol Biol, 313, 451-464(2001)
Non-Patent Document 4: Aizaki, H. et al., Virology, 324, 450-461 (2004)
Non-Patent Document 5: Gao, L. et al., J Virol, 78, 3480-3488 (2004)
Non-Patent Document 6: Fujita, T. et al., J Antibiot (Tokyo), 47, 208-215 (1994)
Non-Patent Document 7: Miyake, Y et al., Biochem Biophys Res Commun, 211, 396-403 (1995)
Non-Patent Document 8: Simons, K. and Ikonen, E., Nature, 387, 569-572 (1997)
Non-Patent Document 9: Sakamoto, H. et al., Nat Chem Biol, 1, 333-337 (2005)

### Disclosure of the Invention

### [Problems to be Solved by the Invention]

The present invention was achieved in view of the circumstances described above. An objective of the present invention is to provide pharmaceutical compositions for treating or preventing HCV infection, which comprise in combination an interferon and a compound that blocks a process of sphingomyelin biosynthesis. Another objective of the present invention is to provide methods for treating or preventing HCV infection, which comprise the step of administering to subjects an interferon and a compound that blocks a process of sphingomyelin biosynthesis.

### [Means for Solving the Problems]

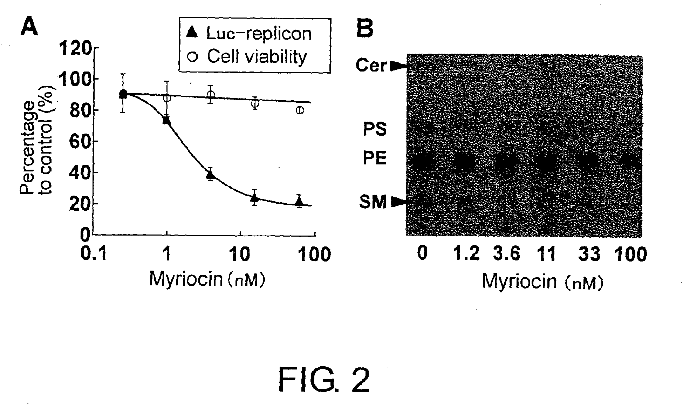

To achieve the above objectives, first, the present inventors investigated the anti-HCV activity and cytotoxicity of myriocin using the HCV subgenomic replicon cell FLR3-1. The result showed that myriocin administration significantly reduced the activity of HCV replication in a dose dependent manner while it had no influence on the cell viability and cell growth (Fig. 2A).

Next, the *de novo* biosynthesis of sphingolipids by FLR3-1 cells in the presence of myriocin was monitored to investigate the correlation between the sphingolipid metabolites and HCV replication. The result showed that the production of ceramide and sphingomyelin was inhibited in a dose-dependent manner while the production of the sphingolipid metabolites, phosphatidylethanolamine and phosphatidylserine, was not affected (Fig. 2B). Furthermore, the anti-HCV effect of myriocin was examined in the presence of intermediates of the sphingolipid biosynthetic pathway to confirm whether or not the suppression of HCV subgenomic replicon replication was caused by depletion of sphingolipids. The result demonstrates that the suppression of HCV replication by myriocin is attributed to the suppression of sphingolipid biosynthesis.

Next, the anti-HCV effect of myriocin and/or interferon was examined using chimeric mice having humanized liver infected with HCV genotype 1a or 1b. Specifically, myriocin and/or interferon was administered to the chimeric mice, and the HCV RNA level in their sera and liver was detected. The results demonstrate that myriocin suppresses the HCV replication without affecting h-Alb expressed from the humanized liver. They also demonstrate that the combined use of myriocin and PEG-IFN synergistically suppresses the HCV replication while it results in mild liver damage.

Furthermore, the anti-HCV effect of interferon and the compound represented by formula (III) was examined by the same method using chimeric mice infected with HCV genotype 1b. The results demonstrate that the administration of the compound of formula (III) also suppresses the HCV replication, and the combination of PEG-IFN and the compound of formula (III) synergistically suppresses the HCV replication.

Thus, the present inventors discovered that myriocin and the compound of formula (III), which are SPT inhibitors, can be used as novel therapeutic agents for HCV infection, and that the combined use of the SPT inhibitor and interferon produced a stronger inhibiting effect on HCV replication, thereby completed the present invention.

More specifically, the present invention provides:
(1) a pharmaceutical composition for treating or preventing HCV infection, which comprises in combination an interferon and a compound that blocks a process of sphingomyelin biosynthesis;
(2) the pharmaceutical composition of (1), wherein the pharmaceutical composition for treating or preventing HCV infection is a combination agent;
(3) the pharmaceutical composition of (1), wherein the interferon and the compound that blocks a process of sphingomyelin biosynthesis are used in combination;
(4) the pharmaceutical composition of (3), wherein the interferon and the compound that blocks a process of sphingomyelin biosynthesis are administered simultaneously or successively;
(5) the pharmaceutical composition of (3), wherein the interferon and the compound that blocks a process of sphingomyelin biosynthesis are administered separately;
(6) a pharmaceutical composition for treating or preventing HCV infection, which comprises as an active ingredient a compound that blocks a process of sphingomyelin biosynthesis, wherein the composition is used in combination with an interferon;
(7) a pharmaceutical composition for treating or preventing HCV infection, which comprises as an active ingredient a compound that blocks a process of sphingomyelin biosynthesis, wherein the composition is administered simultaneously with an interferon;
(8) a pharmaceutical composition for treating or preventing HCV infection, which comprises as an active ingredient a compound that blocks a process of sphingomyelin biosynthesis, wherein the composition is administered before or after administration of an interferon;
(9) a pharmaceutical composition for treating or preventing HCV infection, which comprises an interferon as an active ingredient, wherein the composition is used in combination with a compound that blocks a process of sphingomyelin biosynthesis;
(10) a pharmaceutical composition for treating or preventing HCV infection, which comprises an interferon as an active ingredient, wherein the composition is administered simultaneously with a compound that blocks a process of sphingomyelin biosynthesis;
(11) a pharmaceutical composition for treating or preventing HCV infection, which comprises an interferon as an active ingredient, wherein the composition is administered before or after administration of a compound that blocks a process of sphingomyelin biosynthesis;
(12) the pharmaceutical composition of any one of (1) to (11), wherein the compound that blocks a process of sphingomyelin biosynthesis blocks a process of biosynthesis of sphingomyelin from palmitoyl CoA;
(13) the pharmaceutical composition of any one of (1) to (11), wherein the compound that blocks a process of sphingomyelin biosynthesis is:
   (a) a compound that inhibits the enzymatic activity of a serine palmitoyltransferase involved in the biosynthesis of 3-ketodihydrosphingosine from palmitoyl CoA; or
   (b) a compound that suppresses the expression of a serine palmitoyltransferase;
(14) the pharmaceutical composition of (13), wherein the compound that inhibits the enzymatic activity of a serine palmitoyltransferase is myriocin, sphingofungin, a compound represented by formula (I), or a pharmaceutically acceptable salt thereof: 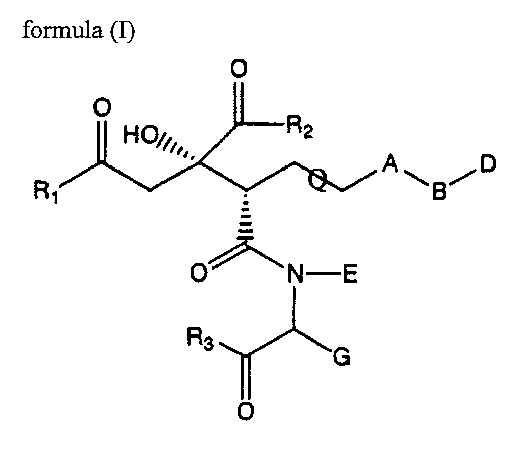 wherein
   A represents -(CH₂)ₙ-, wherein n represents an integer from 0 to 10;
   B represents -CH₂-, -(C=O)-, -CH(OH)-, -CH(NH₂)-, or -C(=NOR)-, wherein R represents a hydrogen atom, linear or branched alkyl group having 1 to 8 carbon atoms, which is optionally substituted by an amino group which is optionally mono- or di-substituted by a linear or branched alkyl group having 1 to 4 carbon atoms;
   D represents -(CH₂)ₘ-R', wherein m represents an integer from 0 to 10, and R' represents a hydrogen atom, linear or branched alkyl group, linear or branched alkynyl group, linear or branched alkenyl group, cycloalkyl group, cycloalkenyl group, optionally substituted heterocyclic group, optionally substituted aryl group, optionally substituted heteroaryl group, -OX group (wherein X represents a hydrogen atom, linear or branched alkyl group, linear or branched alkynyl group, linear or branched alkenyl group, cycloalkyl group, or optionally substituted aryl group), or halogen atom;
   E represents a hydrogen atom, or linear or branched alkyl group;
   G represents -(CH₂)ₚ-J, wherein p represents an integer from 0 to 4, and J represents a hydrogen, OH group, SH group, methylthio group, carboxyl group, carbamoyl group, amino group, guanidino group, linear or branched alkyl group, cycloalkyl group, linear or branched alkynyl group, linear or branched alkenyl group, optionally substituted aryl group, optionally substituted heterocyclic group, or optionally substituted heteroaryl group;
   bond Q represents a single bond or a double bond; and
   R¹, R², and R³ are the same or different and each represent a hydroxyl group, amino group (which is optionally mono- or di-substituted by a linear or branched alkyl group having 1 to 4 carbon atoms), -OL, linear or branched alkyl group, linear or branched alkenyl group, or linear or branched alkynyl group, wherein L represents a linear or branched alkyl group, linear or branched alkenyl group, or linear or branched alkynyl group;
(15) the pharmaceutical composition of (13), wherein the compound that inhibits the enzymatic activity of a serine palmitoyltransferase is a compound represented by any one of formulae (II) to (XII), or a pharmaceutically acceptable salt thereof: 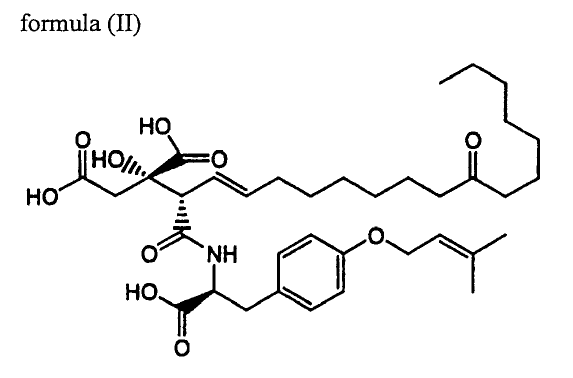 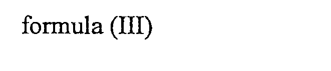 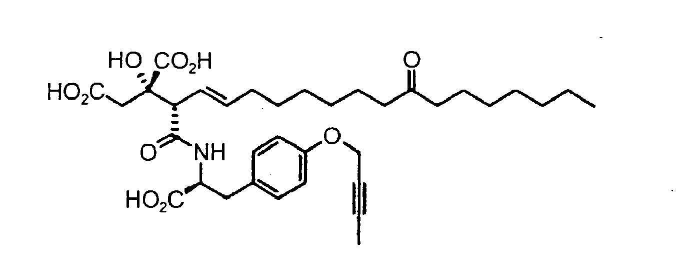 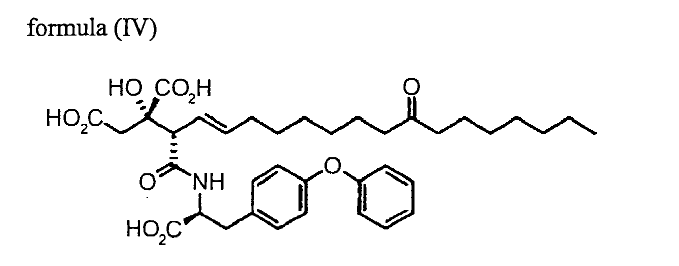 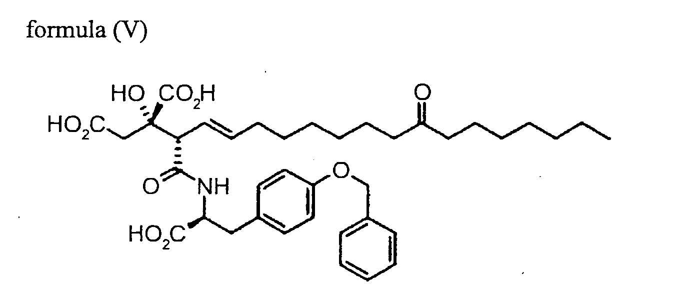 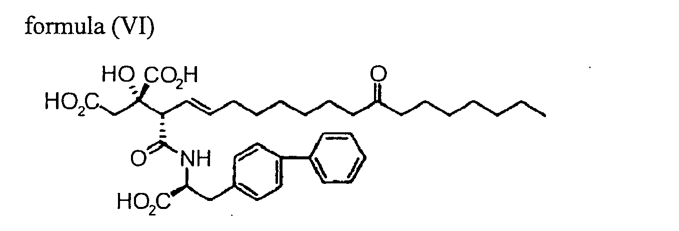 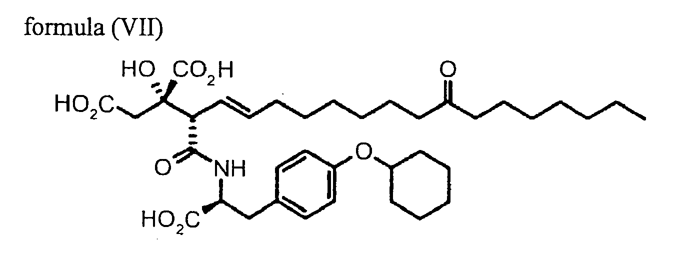 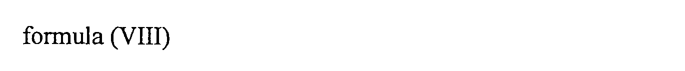 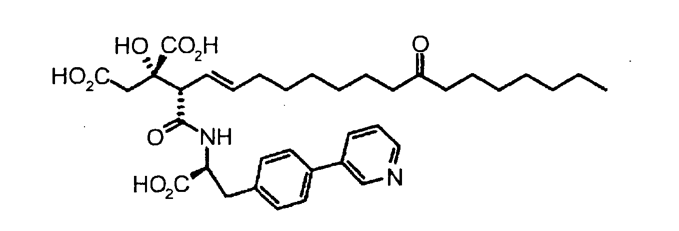 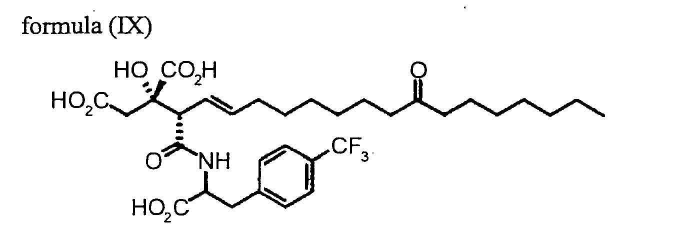 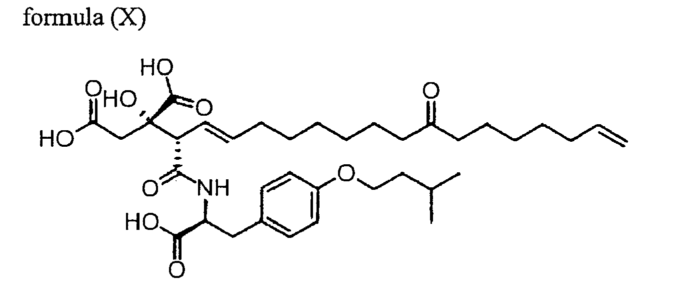 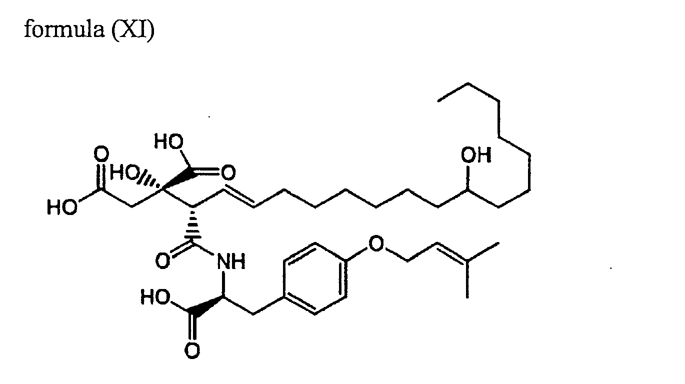 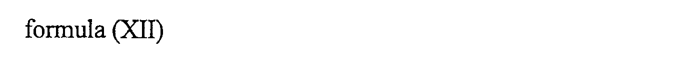 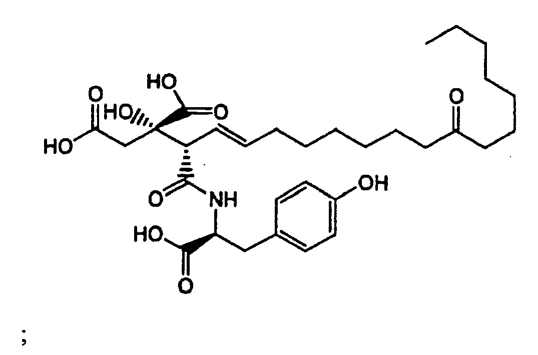
(16) the pharmaceutical composition of (13), wherein the compound that suppresses the expression of a serine palmitoyltransferase is:
   (a) an RNA complementary to a transcript of DNA encoding a serine palmitoyltransferase; or
   (b) an RNA having ribozyme activity to specifically cleave a transcript of DNA encoding a serine palmitoyltransferase;
(17) the pharmaceutical composition of any one of (1) to (11), wherein the interferon is a pegylated interferon;
(18) the pharmaceutical composition of any one of (1) to (11), wherein the HCV infection is type C hepatitis, cirrhosis, hepatic fibrosis, or hepatic cancer;
(19) a method for treating or preventing HCV infection, which comprises the step of administering to a subject an interferon and a compound that blocks a process of sphingomyelin biosynthesis;
(20) the method of (19), wherein the interferon and the compound that blocks a process of sphingomyelin biosynthesis are simultaneously administered to the subject;
(21) the method of (19), wherein the interferon and the compound that blocks a process of sphingomyelin biosynthesis are separately administered to the subject;
(22) the method of (19), wherein the compound that blocks a process of sphingomyelin biosynthesis is a compound that blocks a process of biosynthesis of sphingomyelin from palmitoyl CoA;
(23) the method of (19), wherein the compound that blocks a process of sphingomyelin biosynthesis is:
   (a) a compound that inhibits the enzymatic activity of a serine palmitoyltransferase involved in the biosynthesis of 3-ketodihydrosphingosine from palmitoyl CoA; or
   (b) a compound that suppresses the expression of a serine palmitoyltransferase;
(24) the method of (23), wherein the compound that inhibits the enzymatic activity of a serine palmitoyltransferase is myriocin, sphingofungin, a compound represented by formula (1), or a pharmaceutically acceptable salt thereof: 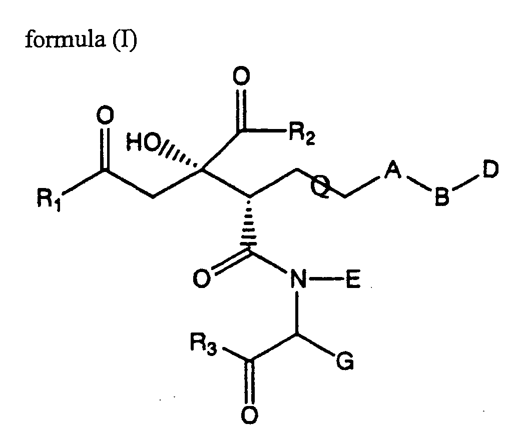 wherein
   A represents -(CH₂)ₙ-, wherein n represents an integer from 0 to 10;
   B represents -CH₂-, -(C=O)-, -CH(OH)-, -CH(NH₂)-, or -C(=NOR)-, wherein R represents a hydrogen atom, linear or branched alkyl group having 1 to 8 carbon atoms, which is optionally substituted by an amino group which is optionally mono- or di-substituted by a linear or branched alkyl group having 1 to 4 carbon atoms;
   D represents -(CH₂)ₘ-R', wherein m represents an integer from 0 to 10, and R' represents a hydrogen atom, linear or branched alkyl group, linear or branched alkynyl group, linear or branched alkenyl group, cycloalkyl group, cycloalkenyl group, optionally substituted heterocyclic group, optionally substituted aryl group, optionally substituted heteroaryl group, -OX group (wherein X represents a hydrogen atom, linear or branched alkyl group, linear or branched alkynyl group, linear or branched alkenyl group, cycloalkyl group, or optionally substituted aryl group), or a halogen atom;
   E represents a hydrogen atom, or linear or branched alkyl group;
   G represents -(CH₂)ₚ-J, wherein p represents an integer from 0 to 4, and J represents a hydrogen, OH group, SH group, methylthio group, carboxyl group, carbamoyl group, amino group, guanidino group, linear or branched alkyl group, cycloalkyl group, linear or branched alkynyl group, linear or branched alkenyl group, optionally substituted aryl group, optionally substituted heterocyclic group, or optionally substituted heteroaryl group;
   bond Q represents a single bond or a double bond; and
   R¹, R², and R³ are the same or different and each represent a hydroxyl group, amino group (which is optionally mono- or di-substituted by a linear or branched alkyl group having 1 to 4 carbon atoms), -OL, linear or branched alkyl group, linear or branched alkenyl group, or linear or branched alkynyl group, wherein L represents a linear or branched alkyl group, linear or branched alkenyl group, or linear or branched alkynyl group;
(25) the method of (23), wherein the compound that inhibits the enzymatic activity of a serine palmitoyltransferase is a compound represented by any one of formulae (II) to (XII), or a pharmaceutically acceptable salt thereof: 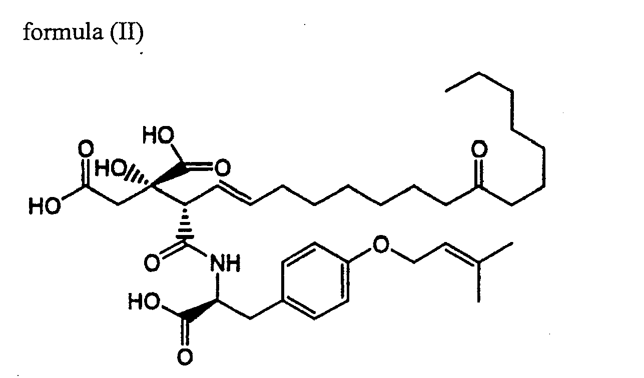 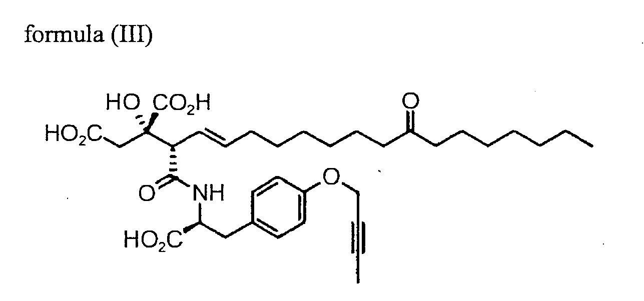 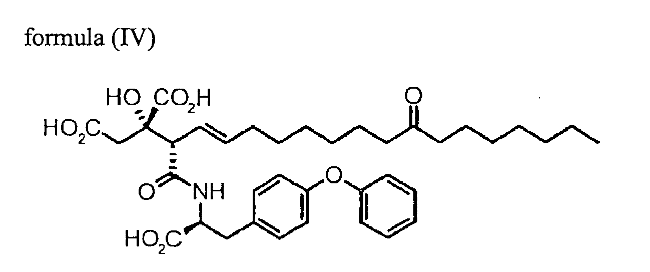 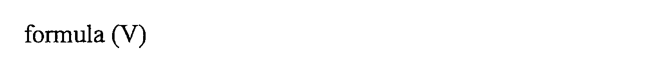 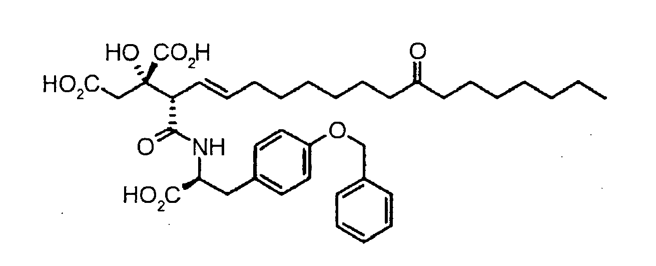 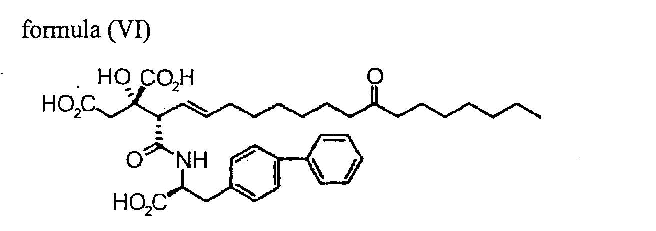 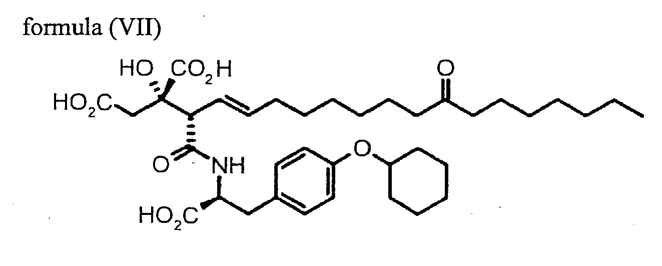 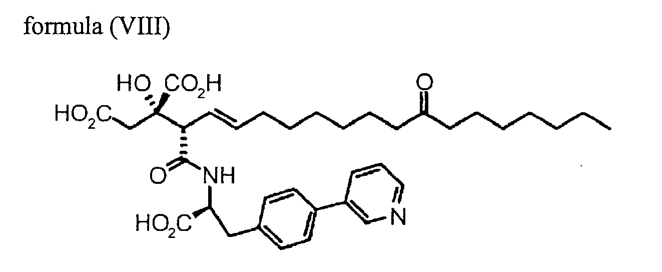 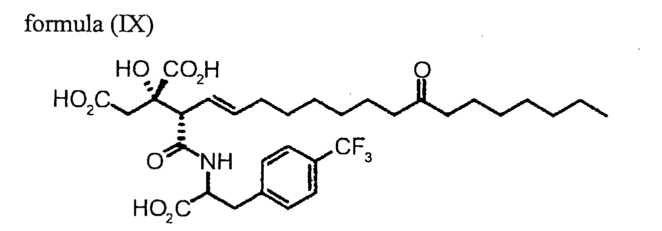 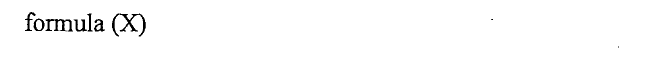 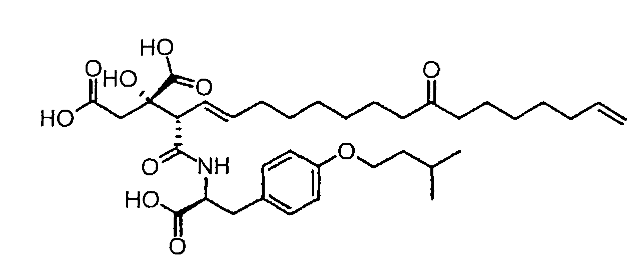 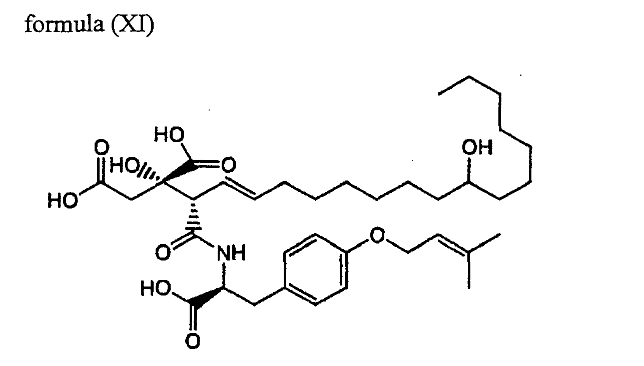 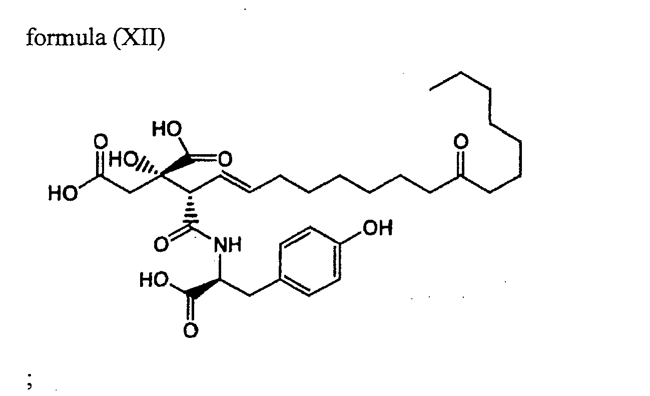
(26) the method of (23), wherein the compound that suppresses the expression of a serine palmitoyltransferase is:
   (a) an RNA complementary to a transcript of a DNA encoding a serine palmitoyltransferase; or
   (b) an RNA having ribozyme activity to specifically cleave a transcript of DNA encoding a serine palmitoyltransferase;
(27) the method of (19), wherein the interferon is a pegylated interferon;
(28) the method of (19), wherein the HCV infection is type C hepatitis, cirrhosis, hepatic fibrosis, or hepatic cancer;
(29) use of an interferon and a compound that blocks a process of sphingomyelin biosynthesis in the preparation of a pharmaceutical composition for treating or preventing HCV infection;
(30) use of a compound that blocks a process of sphingomyelin biosynthesis in the preparation of a pharmaceutical composition for treating or preventing HCV infection which is used in combination with an interferon;
(31) use of a compound that blocks a process of sphingomyelin biosynthesis in the preparation of a pharmaceutical composition for treating or preventing HCV infection which is administered simultaneously with an interferon;
(32) use of a compound that blocks a process of sphingomyelin biosynthesis in the preparation of a pharmaceutical composition for treating or preventing HCV infection which is administered before or after administration of an interferon;
(33) use of an interferon in the preparation of a pharmaceutical composition for treating or preventing HCV infection which is used in combination with a compound that blocks a process of sphingomyelin biosynthesis;
(34) use of an interferon in the preparation of a pharmaceutical composition for treating or preventing HCV infection which is administered simultaneously with a compound that blocks a process of sphingomyelin biosynthesis;
(35) use of an interferon in the preparation of a pharmaceutical composition for treating or preventing HCV infection which is administered before or after administration of a compound that blocks a process of sphingomyelin biosynthesis;
(36) use of any one of (29) to (35), wherein the compound that blocks a process of sphingomyelin biosynthesis is a compound that blocks a process of biosynthesis of sphingomyelin from palmitoyl CoA;
(37) use of any one of (29) to (35), wherein the compound that blocks a process of sphingomyelin biosynthesis is:
   (a) a compound that inhibits the enzymatic activity of a serine palmitoyltransferase involved in the biosynthesis of 3-ketodihydrosphingosine from palmitoyl CoA; or
   (b) a compound that suppresses the expression of a serine palmitoyltransferase;
(38) the use of (37), wherein the compound that inhibits the enzymatic activity of a serine palmitoyltransferase is myriocin, sphingofungin, a compound represented by formula (I), or a pharmaceutically acceptable salt thereof: 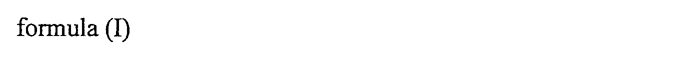 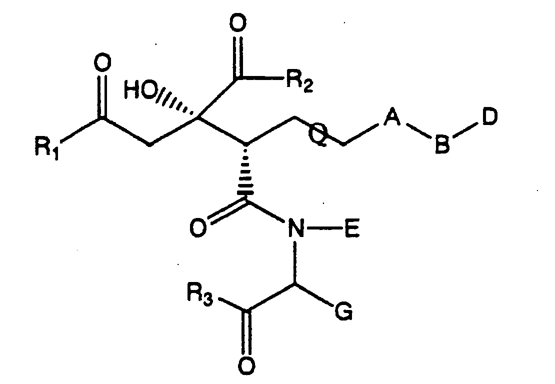 wherein
   A represents -(CH₂)ₙ-, wherein n represents an integer from 0 to 10;
   B represents -CH₂-, -(C=O)-, -CH(OH)-, -CH(NH₂)-, or -C(=NOR)-, wherein R represents a hydrogen atom, linear or branched alkyl group having 1 to 8 carbon atoms, which is optionally substituted by an amino group which is optionally mono- or di-substituted by a linear or branched alkyl group having 1 to 4 carbon atoms;
   D represents -(CH₂)ₘ-R', wherein m represents an integer from 0 to 10, and R' represents a hydrogen atom, linear or branched alkyl group, linear or branched alkynyl group, linear or branched alkenyl group, cycloalkyl group, cycloalkenyl group, optionally substituted heterocyclic group, optionally substituted aryl group, optionally substituted heteroaryl group, -OX group (wherein X represents a hydrogen atom, linear or branched alkyl group, linear or branched alkynyl group, linear or branched alkenyl group, cycloalkyl group, or optionally substituted aryl group), or halogen atom;
   E represents a hydrogen atom, or linear or branched alkyl group;
   G represents -(CH₂)ₚ-J, wherein p represents an integer from 0 to 4; and J represents a hydrogen, OH group, SH group, methylthio group, carboxyl group, carbamoyl group, amino group, guanidino group, linear or branched alkyl group, cycloalkyl group, linear or branched alkynyl group, linear or branched alkenyl group, optionally substituted aryl group, optionally substituted heterocyclic group, or optionally substituted heteroaryl group;
   bond Q represents a single bond or a double bond; and
   R¹, R², and R³ are the same or different and each represent a hydroxyl group, amino group (which is optionally mono- or di-substituted by a linear or branched alkyl group having 1 to 4 carbon atoms), -OL, linear or branched alkyl group, linear or branched alkenyl group, or linear or branched alkynyl group, wherein L represents a linear or branched alkyl group, linear or branched alkenyl group, or linear or branched alkynyl group;
(39) the use of (37), wherein the compound that inhibits the enzymatic activity of a serine palmitoyltransferase is a compound represented by any one of formulae (II) to (XII), or a pharmaceutically acceptable salt thereof: 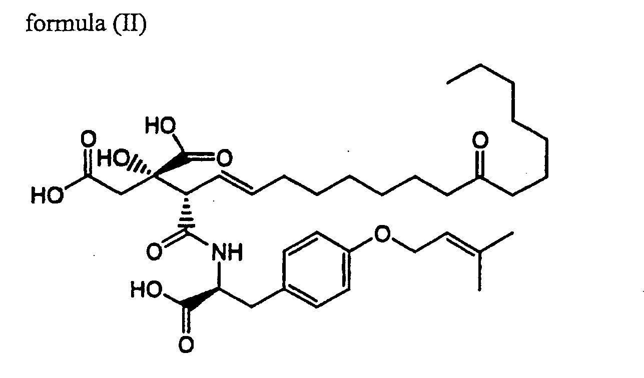 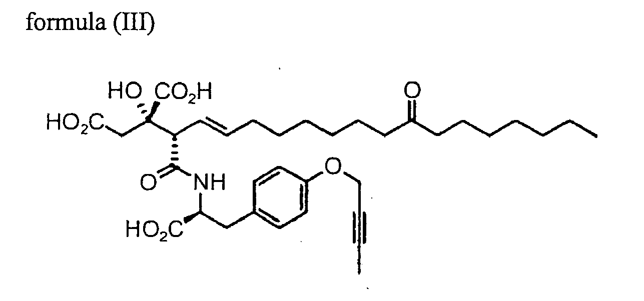 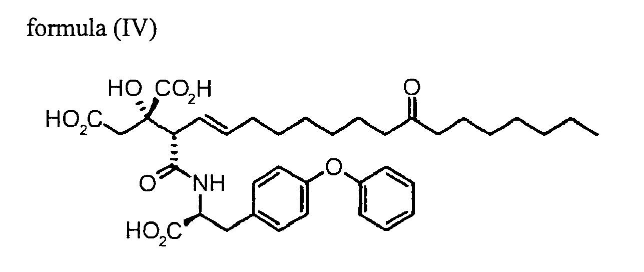 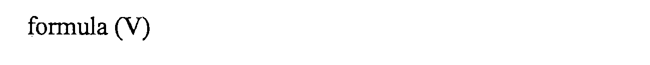 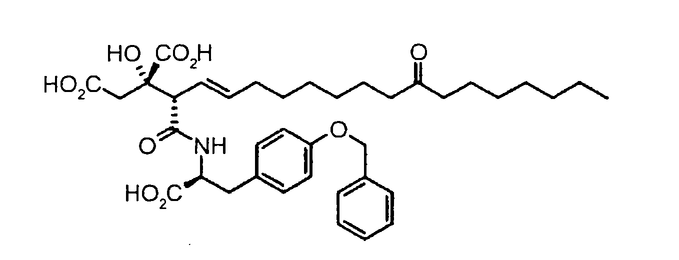 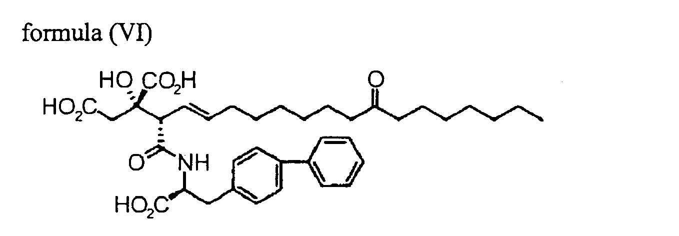 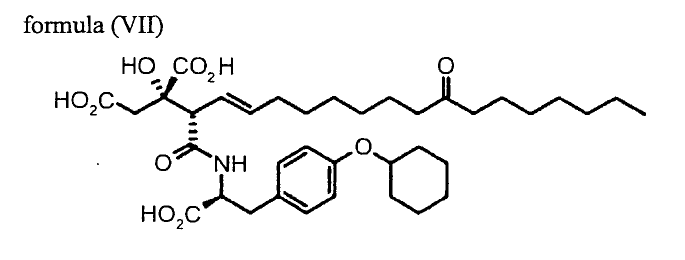 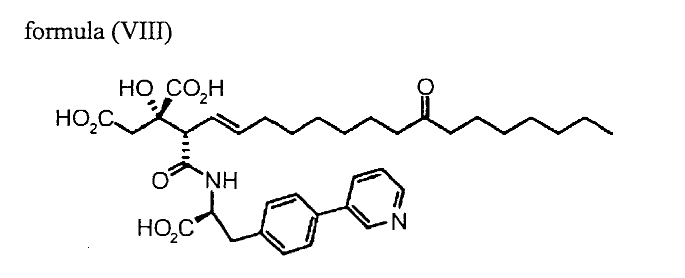 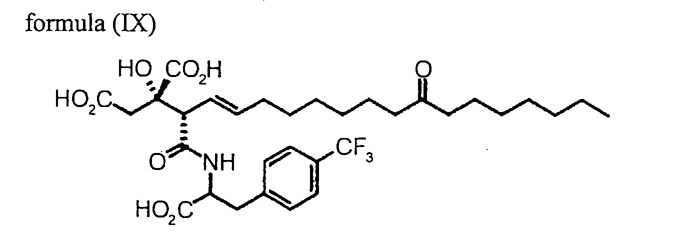 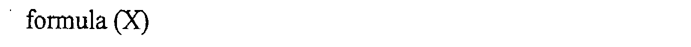 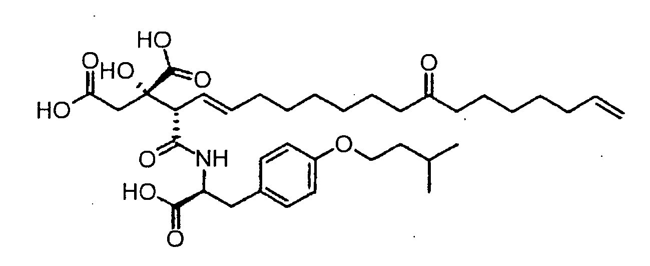 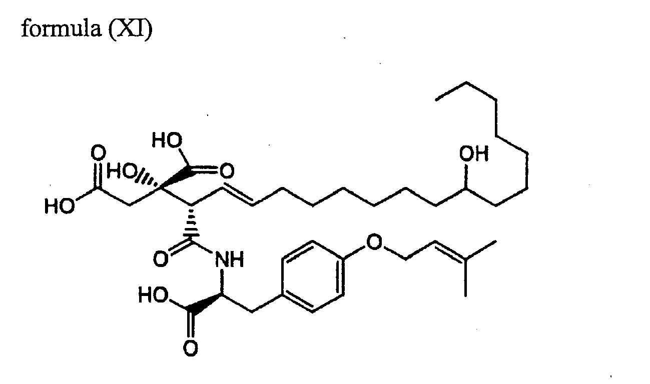 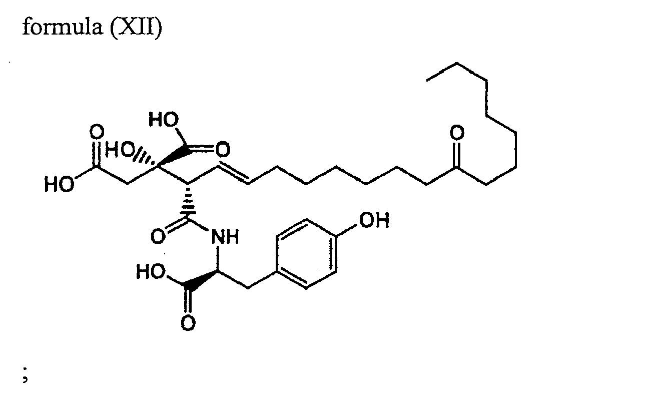
(40) the use of (37), wherein the compound that suppresses the expression of a serine palmitoyltransferase is:
   (a) an RNA complementary to a transcript of DNA encoding a serine palmitoyltransferase; or
   (b) an RNA having ribozyme activity to specifically cleave a transcript of DNA encoding a serine palmitoyltransferase;
(41) the use of any one of (29) to (35), wherein the interferon is a pegylated interferon; and
(42) the use of any one of (29) to (35), wherein the HCV infection is type C hepatitis, cirrhosis, hepatic fibrosis, or hepatic cancer.

### Brief Description of the Drawings

Fig. 1 is a diagram showing the sphingolipid biosynthetic pathway. Serine palmitoyltransferase (SPT) is the primary enzyme in the pathway.
Fig. 2 depicts a graph and a photograph showing the anti-HCV effect of myriocin in HCV replicon cells. (A) is a graph showing luciferase activity and cell viability of FLR3-1 replicon cells in the presence of myriocin. The experiment was carried out at least three times independently. (B) is a photograph showing the result of monitoring *de novo* sphingolipid biosynthesis using TLC. Cer: ceramide, PS: phosphatidylserine, PE: phosphatidylethanolamine, and SM: sphingomyelin.

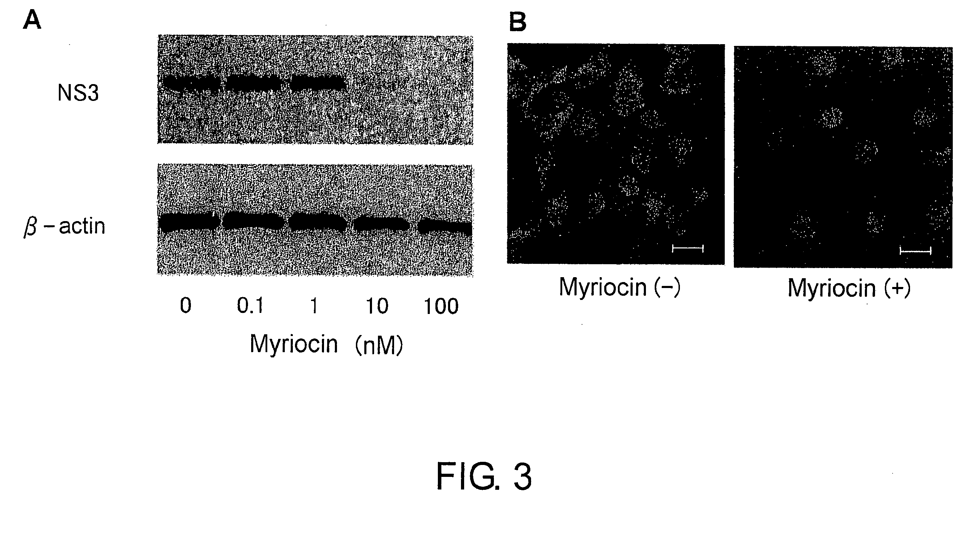

Fig. 3 depicts photographs showing the expression of HCV protein in the presence of myriocin. (A) is a photograph of Western blot, which shows that the expression of NS3 protein was suppressed when myriocin was added to HCV replicon cells. (B) is a photograph of immunostaining, which shows that the expression of NS3 protein was suppressed when myriocin was added to HCV replicon cells.

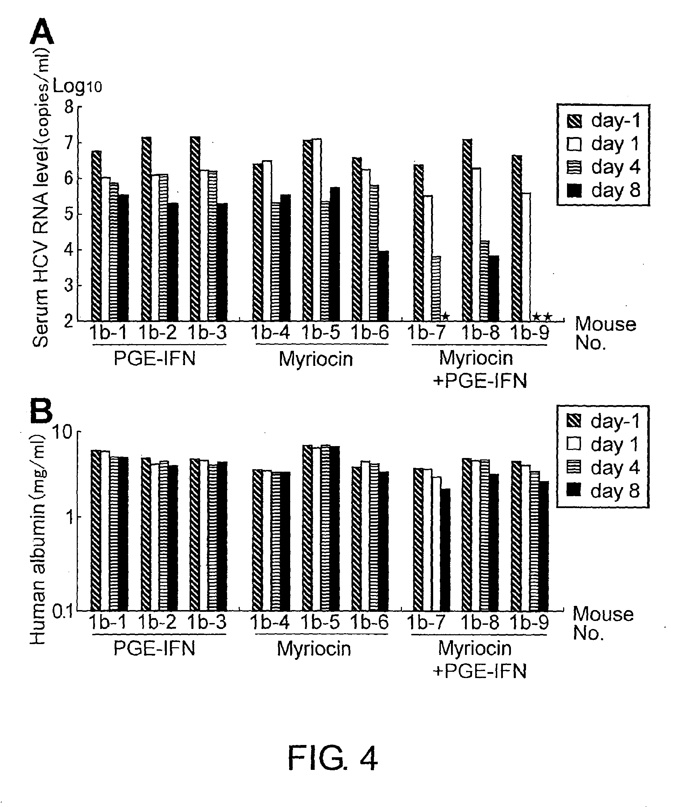

Fig. 4 depicts graphs showing the anti-HCV effect of myriocin in chimeric mice infected with HCV genotype 1b. (A) is a graph showing the level of HCV RNA in the sera of chimeric mice. The stars indicate that HCV RNA was undetectable. (B) is a graph showing the human albumin level in the sera of chimeric mice.

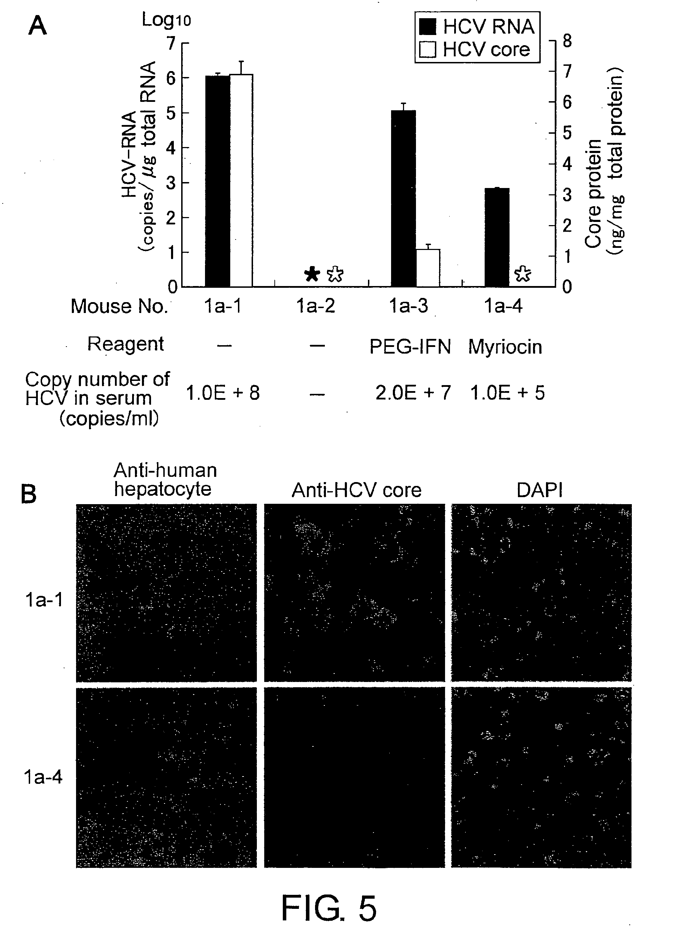

Fig. 5 depicts a graph and a photograph showing the results of detecting HCV RNA and core protein in the livers of myriocin-treated chimeric mice. HCV RNA and core protein were detected in the livers of chimeric mice infected with HCV genotype 1a. 1a-1: non-treated; 1a-2: non-treated and non-infected; 1a-3: PEG-IFN-treated; and 1a-4: myriocin-treated. (A) is a graph showing the copy number of HCV RNAper µg of total RNA and the expression level of HCV core protein per mg of total protein. The stars indicate that HCV RNA or core protein was undetectable. (B) is a photograph showing the results of immunofluorescent staining of HCV core protein and human hepatocytes in the livers of chimeric mice. The staining results for human hepatocytes, HCV core protein, and nuclei are shown from left.

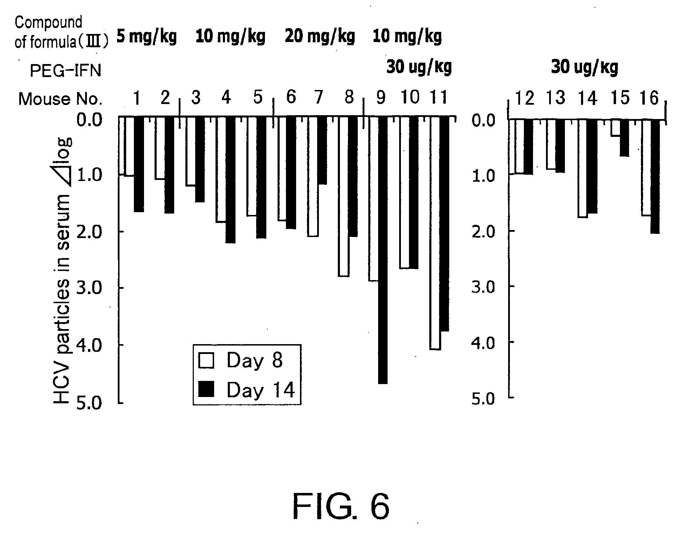

Fig. 6 is a graph showing the anti-HCV effect of the compound of formula (III) in chimeric mice infected with HCV genotype 1b. The serum HCV RNA levels on days 8 and 14 in the chimeric mice of each administration group are shown.

### Best Mode for Carrying Out the Invention

The present invention relates to pharmaceutical compositions for treating or preventing HCV infection, which comprise in combination an interferon and a compound that blocks a process of sphingomyelin biosynthesis.

In the present invention, the "pharmaceutical compositions for treating or preventing HCV infection, which comprise in combination an interferon and a compound that blocks a process of sphingomyelin biosynthesis" may be pharmaceutical compositions in which an interferon and a compound that blocks a process of sphingomyelin biosynthesis are combined in order to administer them simultaneously, separately, or successively in the treatment or prevention of HCV infection. The pharmaceutical compositions of the present invention may be combination agents, in which an interferon and a compound that blocks a process of sphingomyelin biosynthesis are contained in the same pharmaceutical composition. Alternatively, an interferon and a compound that blocks a process of sphingomyelin biosynthesis may be separately contained in different pharmaceutical compositions. Moreover, a pharmaceutical composition comprising an interferon and a pharmaceutical composition comprising a compound that blocks a process of sphingomyelin biosynthesis may be combined as a kit.

Regarding the above-described "pharmaceutical composition for treating or preventing HCV infection", when an interferon and a compound that blocks a process of sphingomyelin biosynthesis are separately contained in different pharmaceutical compositions, the dosage forms of the two separate preparations may be the same or different. For example, either or both of them may be parenteral preparations, injections, drip infusions, or intravenous drip infusions.

The above-described "pharmaceutical composition for treating or preventing HCV infection" also includes compositions in which one or more preparations are further combined with the above-described combination preparation used in the treatment or prevention of HCV infection.

The present invention also relates to pharmaceutical compositions for treating or preventing HCV infection, which comprise as an active ingredient a compound that blocks a process of sphingomyelin biosynthesis, wherein the compositions are used in combination with an interferon. When the pharmaceutical compositions are used in combination with an interferon, the compositions may be administered simultaneously with an interferon, or before or after administration of an interferon.

The present invention also relates to pharmaceutical compositions for treating or preventing HCV infection, which comprise an interferon as an active ingredient, wherein the compositions are used in combination with a compound that blocks a process of sphingomyelin biosynthesis. When an interferon is used in combination with the pharmaceutical compositions, it may be administered simultaneously with the pharmaceutical compositions, or before or after administration of the pharmaceutical compositions.

Examples of the process of sphingomyelin biosynthesis in the present invention include the process of sphingomyelin biosynthesis from palmitoyl CoA (Fig. 1).

In the present invention, the compounds that block a process of sphingomyelin biosynthesis may be any compounds, so long as they directly or indirectly inhibit an *in* vivo reaction involved in the process of sphingomyelin biosynthesis from palmitoyl CoA. Such compounds may be compounds that inhibit the activity of an enzyme involved in the process of sphingomyelin biosynthesis, compounds that inhibit the expression of an enzyme involved in the biosynthesis, or compounds that produce or increase the amount of such inhibitors and thereby indirectly inhibit an enzyme involved in the biosynthesis.

Whether the compounds of the present invention inhibit a process of sphingomyelin biosynthesis can be assessed by measuring their 50% inhibitory concentration (IC₅₀) in the sphingomyelin biosynthesis. The compounds of the present invention may, without limitation, block a process of sphingomyelin biosynthesis with a final IC₅₀ value of preferably 1 µM or less, more preferably 100 nM or less, most preferably 50 nM or less against the normal sphingomyelin biosynthesis. The IC₅₀ value in the sphingomyelin biosynthesis can be determined by those skilled in the art using any methods. Such methods include, for example, a method in which cells are allowed to incorporate [¹⁴C]-serine and its level in the lipid is determined (Nat Chem Biol. 2005 Nov;1 (6):333-7. Epub 2005 Oct 16) and a method in which the activity of the compounds to inhibit the HCV RNA replication is determined by the luciferase assay using HCV subgenomic replicon cells (Example 1; and Nat Chem Biol. 2005 Nov;1(6):333-7. Epub 2005 Oct 16).

In the present invention, examples of enzymes involved in the process of sphingomyelin biosynthesis include serine palmitoyltransferase, 3-ketodihydrosphingosine reductase, dihydrosphingosine-*N*-acyltransferase, dihydroceramide desaturase, and sphingomyelin synthase (Fig. 1), and preferably include serine palmitoyltransferase.

Examples of the compounds of the present invention include compounds that block the process of 3-ketodihydrosphingosine biosynthesis from palmitoyl CoA, which is the initial step in the process of sphingolipid biosynthesis. Examples of such compounds that block this biosynthetic process are compounds that inhibit the enzymatic activity of serine palmitoyltransferase involved in the biosynthesis, or compounds that suppress the expression of serine palmitoyltransferase.

Compounds of the present invention that inhibit the enzymatic activity of serine palmitoyltransferase may be any compounds so long as they inhibit the enzymatic activity

Whether the compounds of the present invention inhibit the enzymatic activity of serine palmitoyltransferase can be assessed by measuring their 50% inhibitory concentration (IC₅₀) against the enzymatic activity of serine palmitoyltransferase. The compounds of the present invention may, without limitation, have an IC₅₀ value of preferably 1 µM or less, more preferably 100 µM or less, most preferably 50 nM or less, against the enzymatic activity of serine palmitoyltransferase. The IC₅₀ value against the enzymatic activity of serine palmitoyltransferase can be determined by those skilled in the art using any methods. Such methods include, for example, a method in which cells are allowed to incorporate [¹⁴C]-serine and its level in the lipid is determined (Nat Chem Biol. 2005 Nov;1(6):333-7. Epub 2005 Oct 16).

The compounds of the present invention that inhibit the enzymatic activity of serine palmitoyltransferase preferably include, for example, myriocin, sphingofungin, the compounds represented by formula (I), and pharmaceutically acceptable salts thereof. 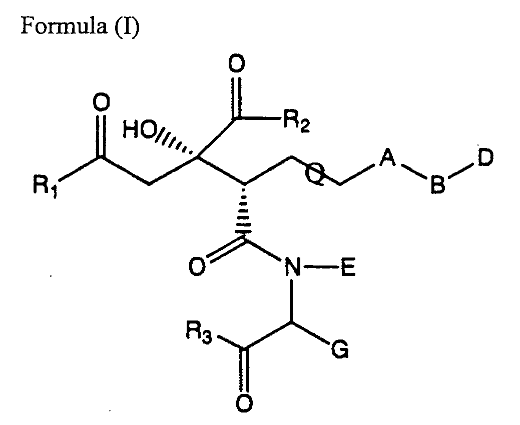 wherein
A represents -(CH₂)ₙ-, wherein n is an integer from 0 to 10;
B represents -CH₂-, -(C=O)-, -CH(OH)-, -CH(NH₂)-, or -C(=NOR)-, wherein R represents a hydrogen atom, or a linear or branched alkyl group of one to eight carbon atoms, which is optionally substituted with an amino group that is optionally mono- or di-substituted with a linear or branched alkyl group of one to four carbon atoms;
D represents -(CH₂)ₘ-R', wherein m is an integer from 0 to 10, and R' represents a hydrogen atom, linear or branched alkyl group, linear or branched alkynyl group, linear or branched alkenyl group, cycloalkyl group, optionally substituted heterocyclic group, optionally substituted aryl group, optionally substituted heteroaryl group, -OX group (wherein X denotes a hydrogen atom, or linear or branched alkyl group, linear or branched alkynyl group, linear or branched alkenyl group, cycloalkyl group, or optionally substituted aryl group), or halogen atom;
E represents a hydrogen atom or a linear or branched alkyl group;
G represents -(CH₂)ₚ-J, wherein p is an integer of 0 to 4, and J represents a hydrogen, OH group, SH group, methylthio group, carboxyl group, carbamoyl group, amino group, guanidino group, linear or branched alkyl group, cycloalkyl group, linear or branched alkynyl group, linear or branched alkenyl group, optionally substituted aryl group, optionally substituted heterocyclic group, or optionally substituted heteroaryl group;
bond Q represents a single bond or a double bond; and
R¹, R², and R³ are the same or different and represent a hydroxyl group, amino group that is optionally mono- or di-substituted with a linear or branched alkyl group of one to four carbon atoms, -OL, linear or branched alkyl group, linear or branched alkenyl group, or linear or branched alkynyl group, wherein L represents a linear or branched alkyl group, linear or branched alkenyl group, or linear or branched alkynyl group.

In the present invention, unless specifically defined herein, the linear or branched alkyl group means a linear or branched hydrocarbon group of one to twelve carbons, and preferably means a linear or branched hydrocarbon group of one to seven carbons. Examples include a methyl group, ethyl group, propyl group, isopropyl group, *n*-butyl group, isobutyl group, *t*-butyl group, pentyl group, and heptyl group. The cycloalkyl group means a cyclic hydrocarbon group of three to eight carbons. Examples include a cyclopentyl group, cyclohexyl group, cycloheptyl group, and cyclohexenyl group. The linear or branched alkenyl group means a linear or branched hydrocarbon group of two to eight carbons, which comprises at least one double bond. Examples include a vinyl group, 1-propenyl group, allyl group, 2-butenyl group, and 2-ethenyl-2-butenyl group. The linear or branched alkynyl group means a linear or branched hydrocarbon group of two to eight carbons, which comprises at least one triple bond. Examples include an ethynyl group, 1-propynyl group, 2-propynyl group, 1-butynyl group, 3-butynyl group, 2-pentynyl group, 3-pentynyl group, 4-pentynyl group, 2-hexynyl group, 4-hexynyl group, 2-decynyl group, and 6,6-dimethyl-hepta-2,4-diyn-1-yl group.

The heterocyclic group described herein means a four- to six-membered monocyclic or seven- to ten-membered bicyclic group (preferably a monocyclic group), which comprises one to four (preferably one or two) heteroatoms that are individually selected from nitrogen, sulfur, and oxygen atoms as ring members, and may comprise at least one double bond. Specific examples include groups derived from pyran, morpholine, tetrahydrofuran, dihydrofuran, tetrahydropyran, dihydropyran, 1,3-dioxane, piperazine, piperidine, thiomorpholine, and such.

The aryl group described herein means a monocyclic or polycyclic hydrocarbon group that has aromaticity. Specific examples include groups derived from benzene, naphthalene, anthracene, and fluorene.

The heteroaryl group described herein means a four- to six-membered monocyclic or seven- to ten-membered bicyclic group (preferably a monocyclic group) which has aromaticity, and comprises one to four (preferably one or two) heteroatoms that are individually selected from nitrogen, sulfur, and oxygen atoms as ring members. Specific examples include groups derived from furan, thiophene, pyrrole, diazole, pyridine, thiazole, imidazole, pyrimidine, indole, quinoline, oxazole, isoxazole, pyrazine, triazole, thiadiazole, tetrazole, and pyrazole.

The aralkyl group described herein means the above-mentioned linear or branched alkyl group substituted with the above-mentioned aryl group, and specific examples include a benzyl group and a phenethyl group.

The heteroarylalkyl group described herein means the above-mentioned linear or branched alkyl group substituted with the above-mentioned heteroaryl group.

The acyl group described herein means the above-mentioned linear or branched alkyl group, aryl group, heteroaryl group, or heterocyclic group that is bonded via a carbonyl group.

The phrase "optionally substituted" described herein, unless particularly defined herein, means that a group may be substituted with a group such as a linear or branched alkyl group, linear or branched alkoxy group, linear or branched alkenyl group, linear or branched alkenyloxy group, linear or branched alkynyl group, linear or branched alkynyloxy group, cycloalkyl group, cycloalkyloxy group, cyano group, nitro group, trifluoromethyl group, trifluoromethoxy group, halogen atom, aryl group, aryloxy group, heteroaryl group, heteroaryloxy group, aralkyl group, aralkyloxy group, amino group (which is optionally mono- or di-substituted with a linear or branched alkyl group), acyl group, linear or branched alkylsulfonyl group, carbamoyl group, linear or branched alkylthio group, carboxyl group, linear or branched alkylcarbonyl group, formyl group, and aminosulfonyl group. The aryl and heteroaryl moieties included in these substituent groups may be further mono-, di-, or tri-substituted with a halogen atom, linear or branched alkyl group, linear or branched alkoxy group, linear or branched alkenyl group, linear or branched alkenyloxy group, linear or branched alkynyl group, linear or branched alkynyloxy group, cycloalkyl group, cycloalkyloxy group, cyano group, nitro group, trifluoromethyl group, trifluoromethoxy group, halogen atom, aryl group, aryloxy group, heteroaryl group, aralkyl group, aralkyloxy group, amino group that is optionally mono- or di-substituted with a linear or branched alkyl group, acyl group, linear or branched alkylsulfonyl group, linear or branched alkoxy group, carbamoyl group, linear or branched alkylthio group, carboxyl group, linear or branched alkylcarbonyl group, formyl group, aminosulfonyl group, and such.

Furthermore, pharmaceutically acceptable salts of the compounds represented by formula (I) can be produced by contacting the compounds with an acid or base that can be used in producing pharmaceuticals. The salts are not particularly limited as long as they are pharmaceutically acceptable, and include, for example, salts with mineral acids such as hydrochloric acid, sulfuric acid, nitric acid, phosphoric acid, and hydrobromic acid; salts with organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid; and salts with alkali metals and alkali earth metals such as sodium, potassium, and calcium.

Furthermore, the salts include hydrates and solvates that may be formed by the compounds. When a compound represented by formula (I) is obtained in a free form, it can be converted into salts or their hydrates or solvates that may be formed by the compound, according to conventional methods.

The compounds represented by formula (I) of the present invention can be synthesized by methods described in WO2004/071503, WO2005/005372, and WO2006/016657.

Preferred examples of the compounds represented by formula (I) of the present invention include the following compounds:

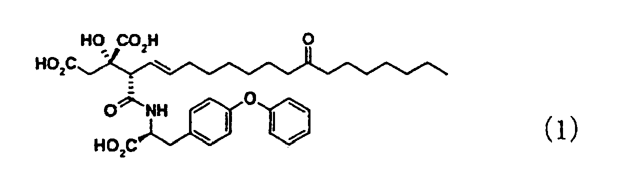

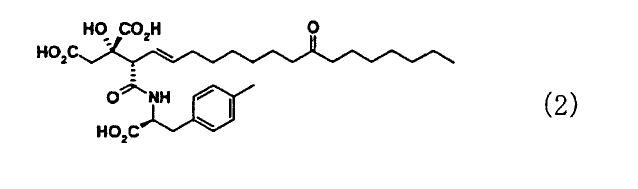

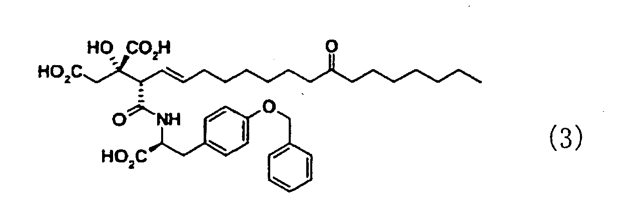

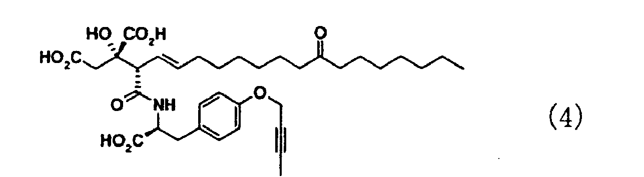

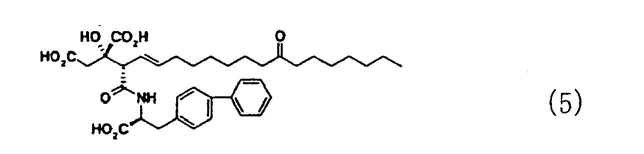

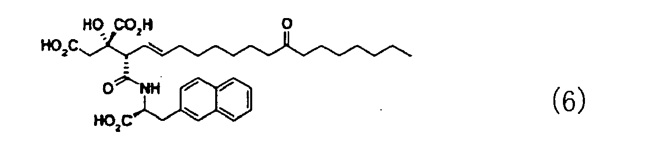

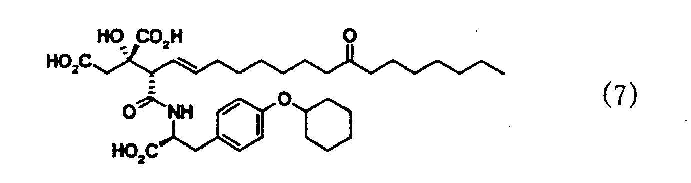

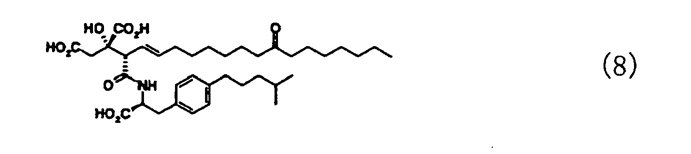

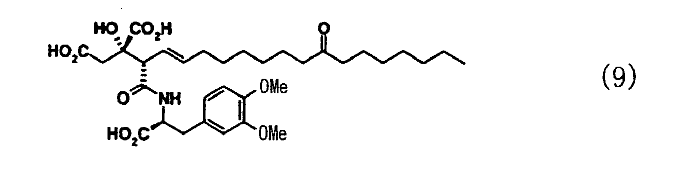

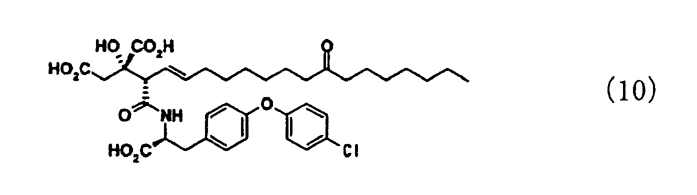

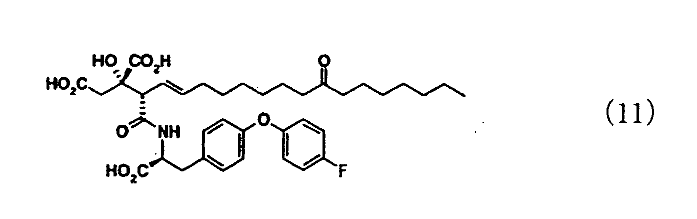

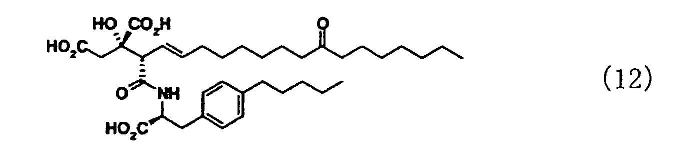

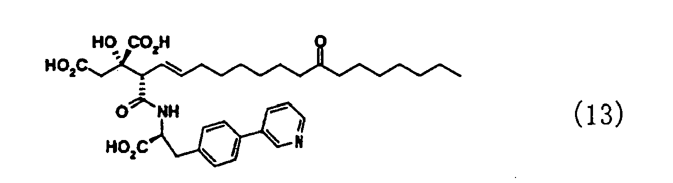

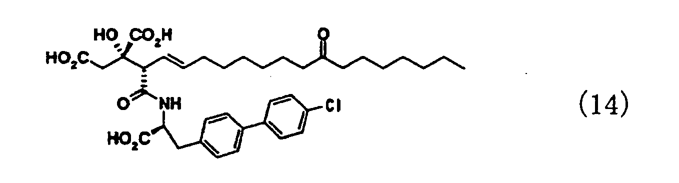

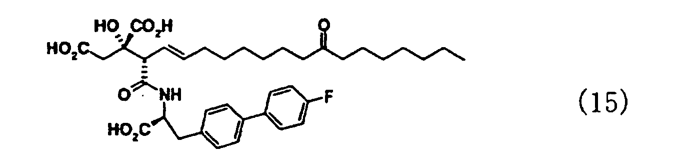

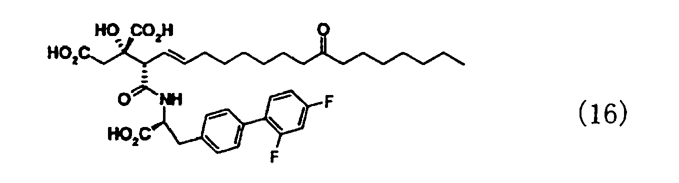

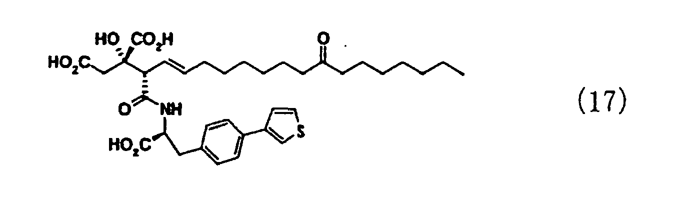

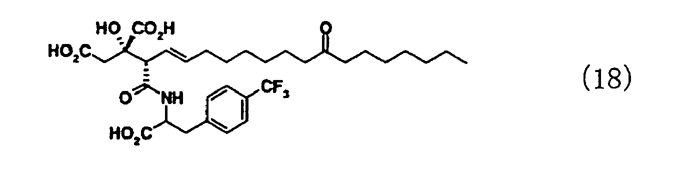

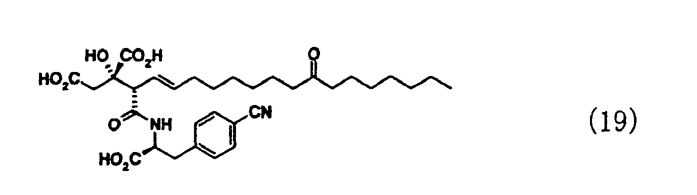

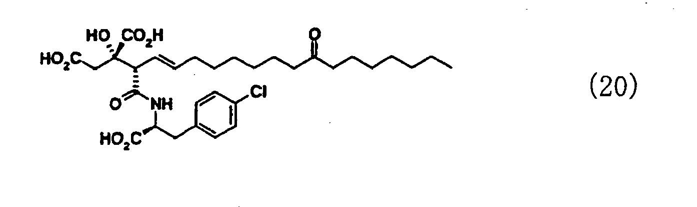

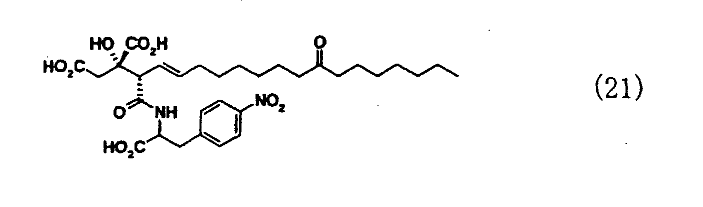

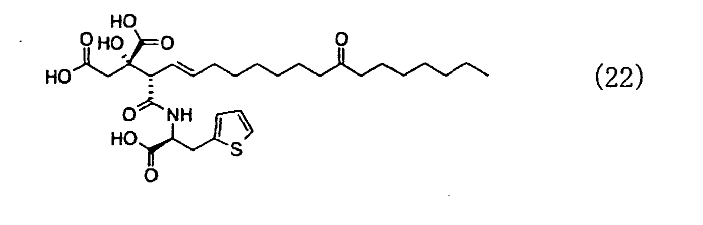

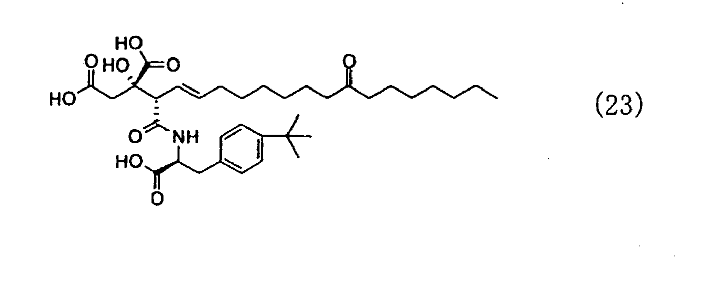

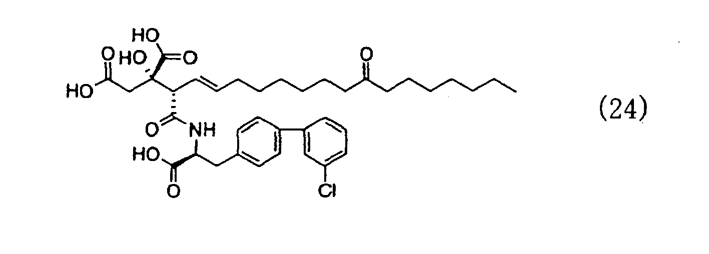

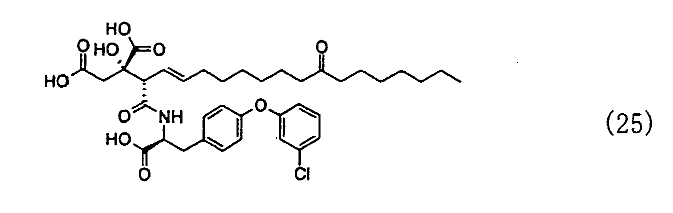

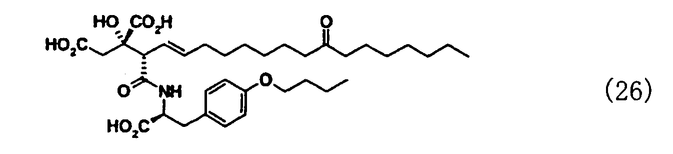

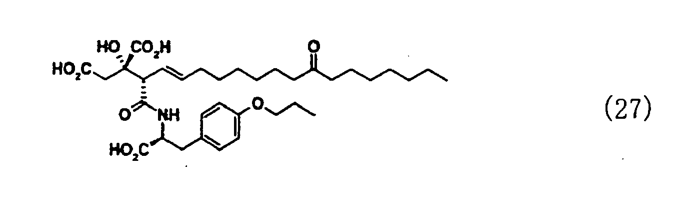

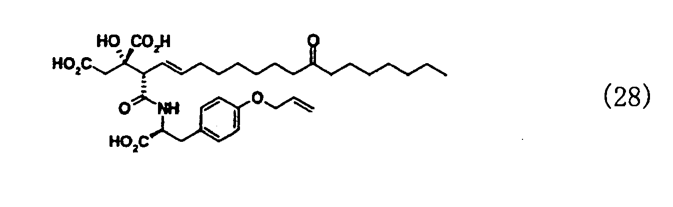

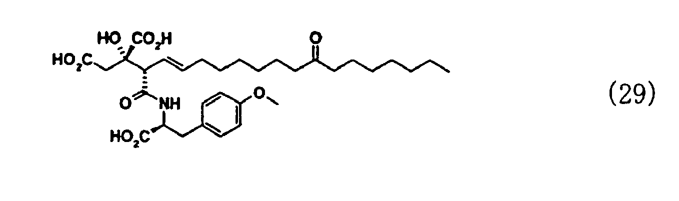

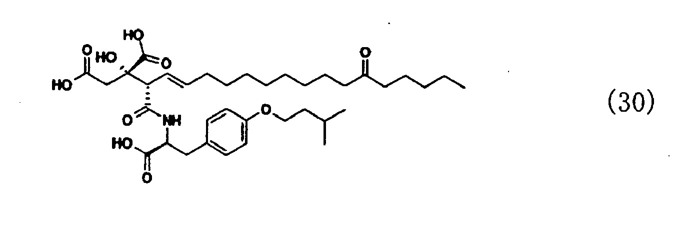

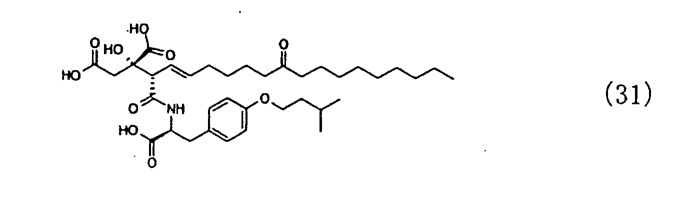

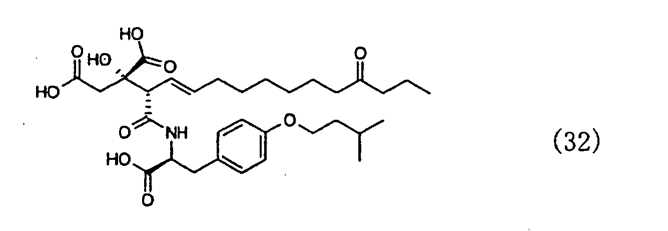

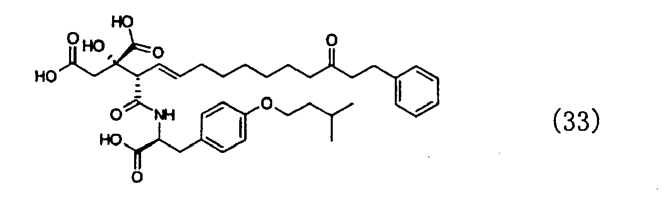

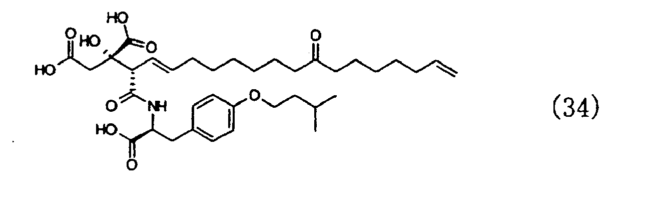

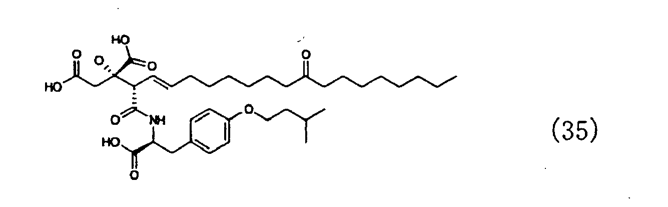

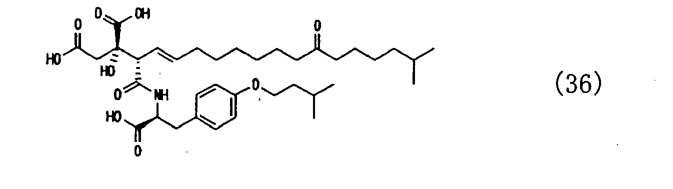

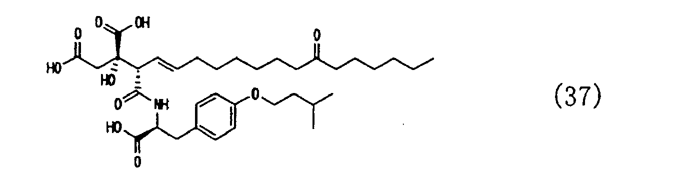

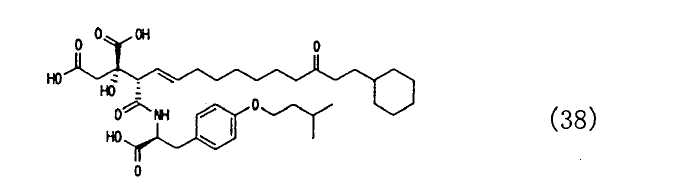

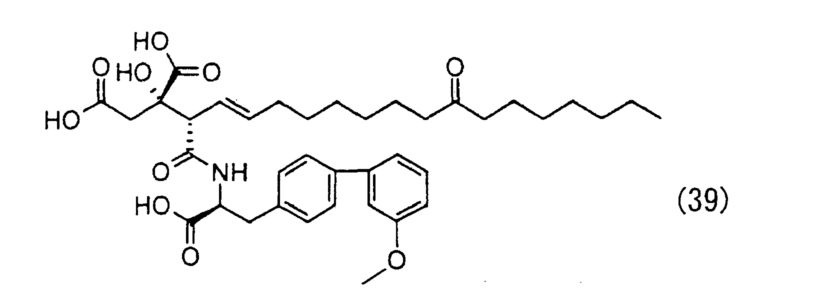

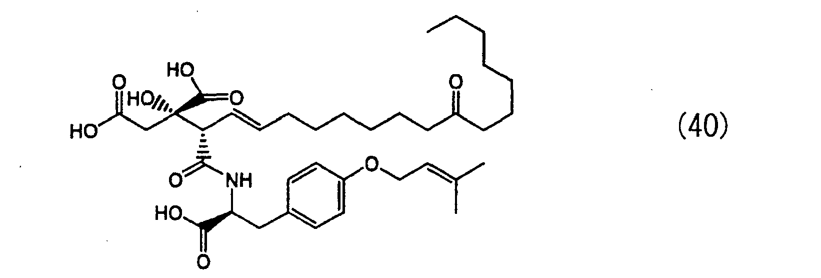

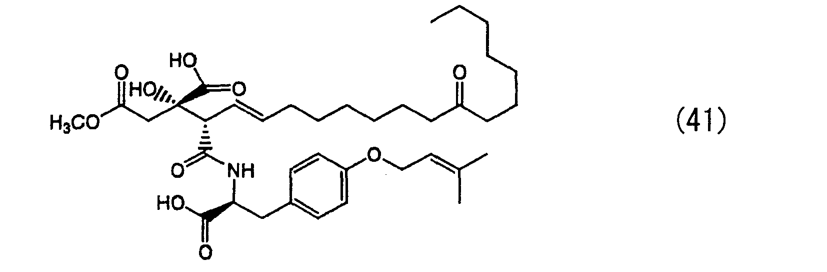

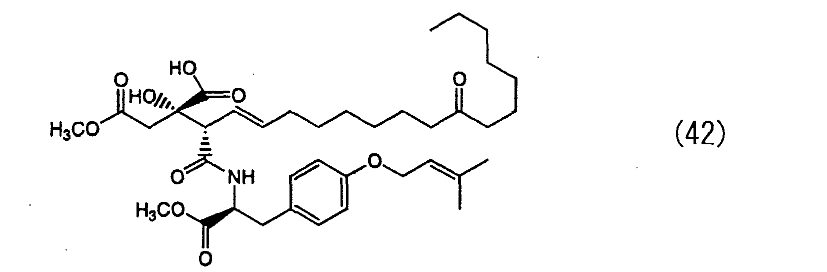

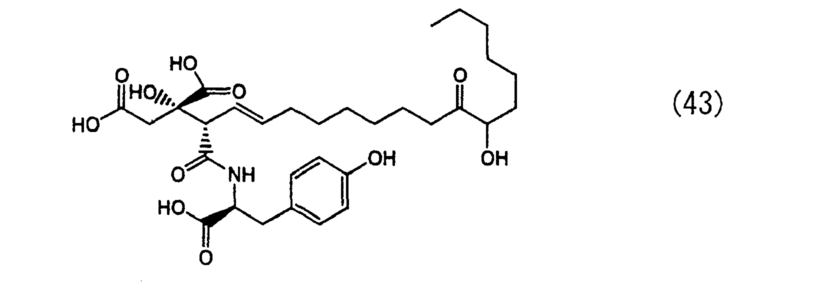

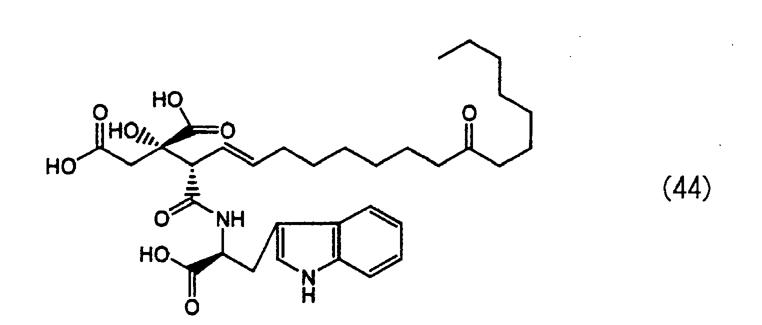

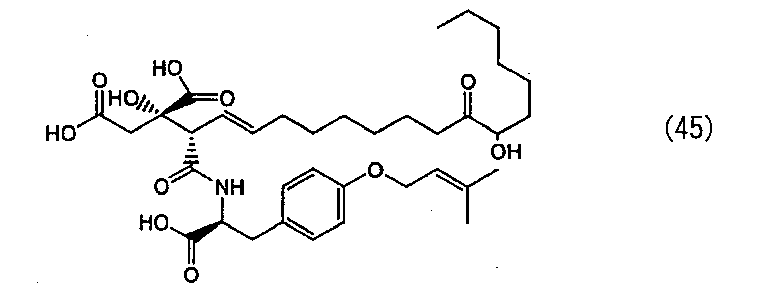

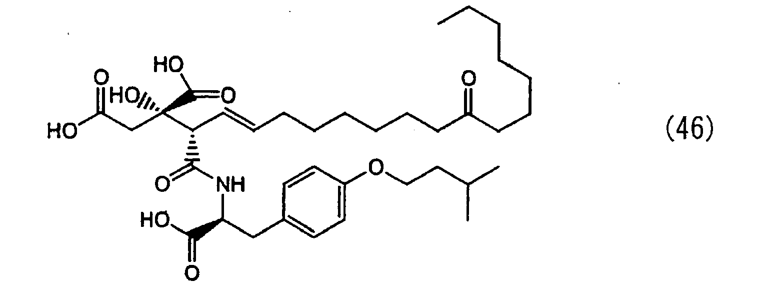

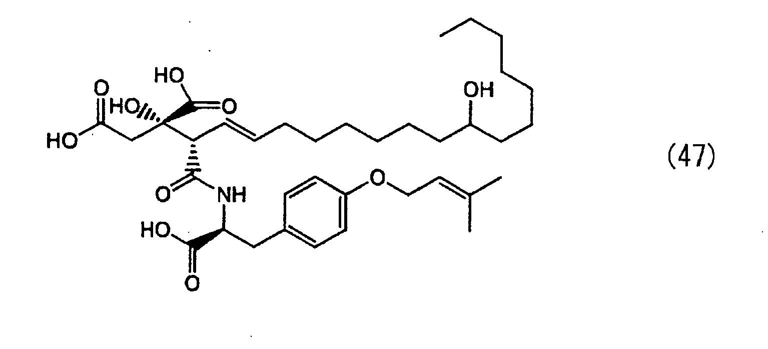

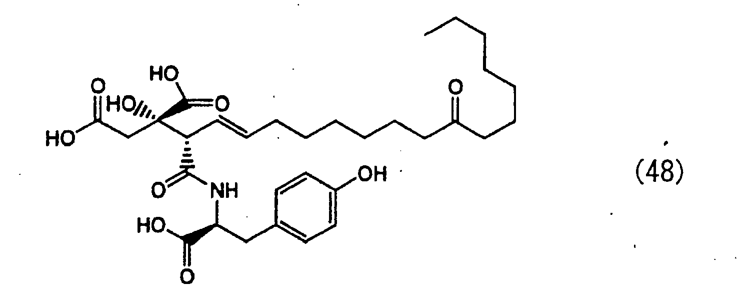

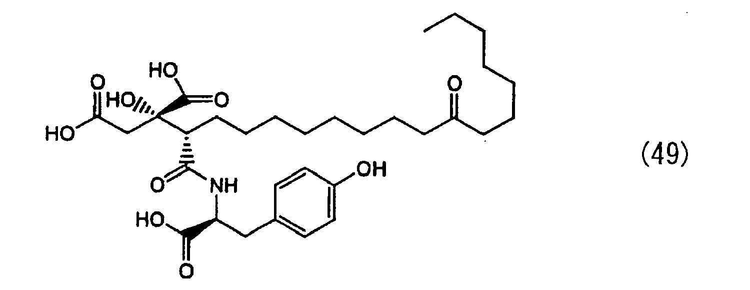

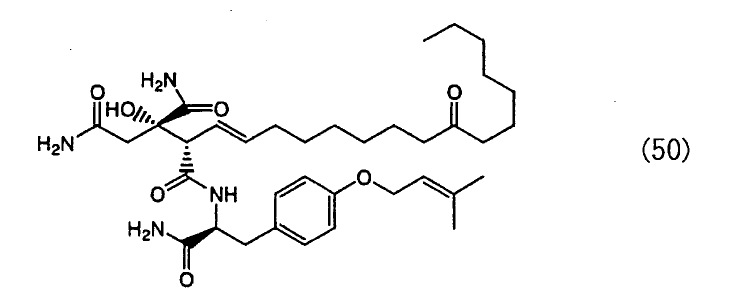

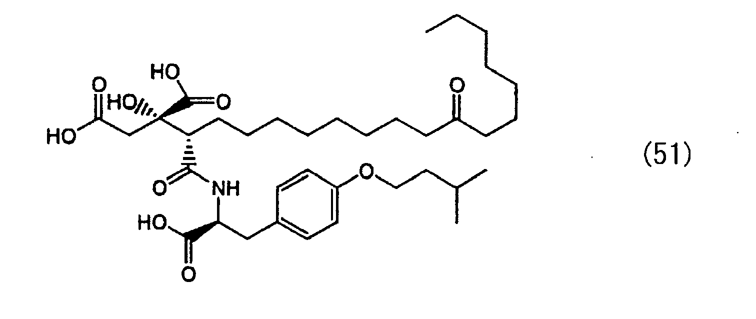

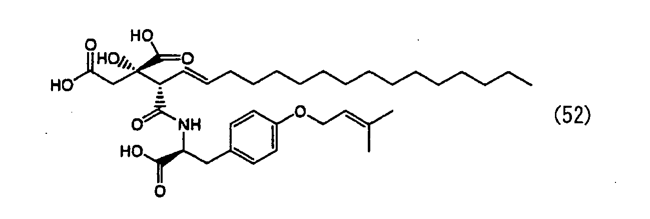

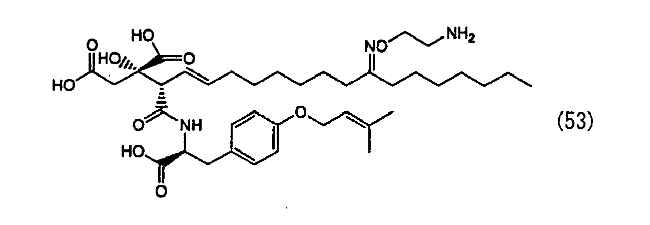

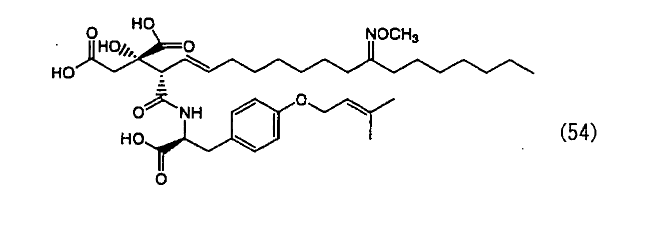

More preferred examples of the compounds represented by formula (I) of the present invention include the compounds represented by formulae (II) to (XII).

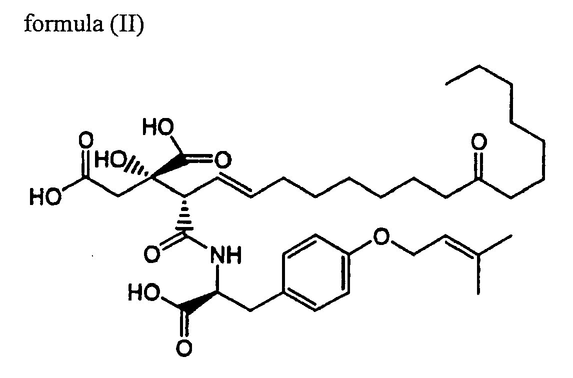

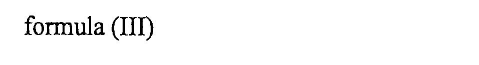

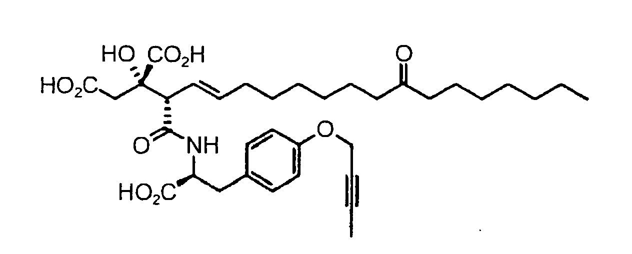

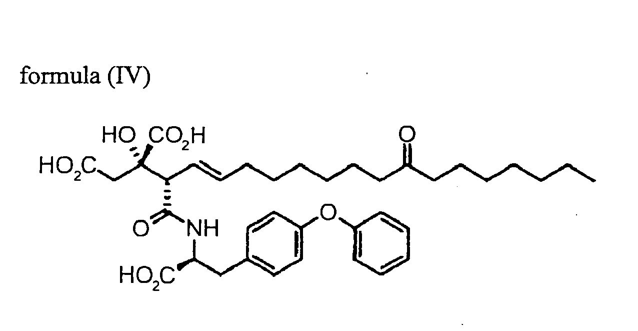

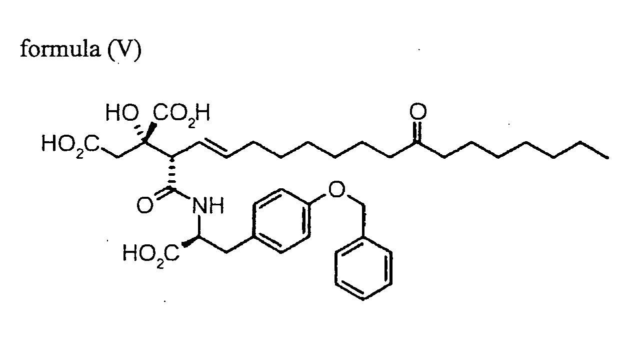

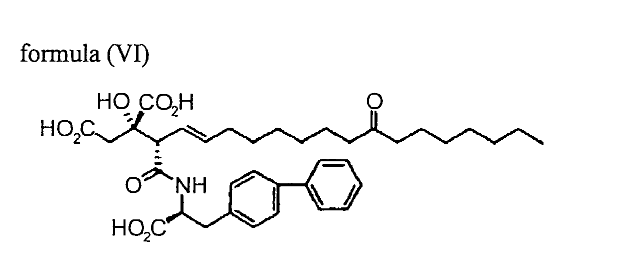

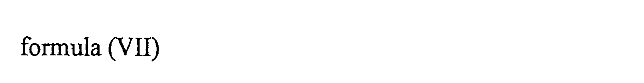

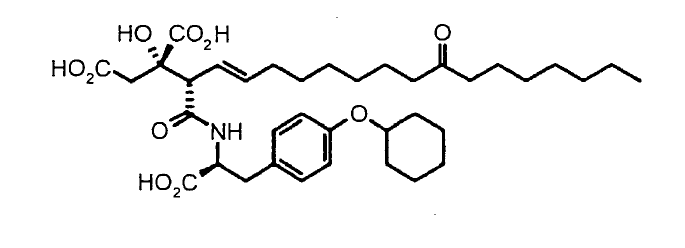

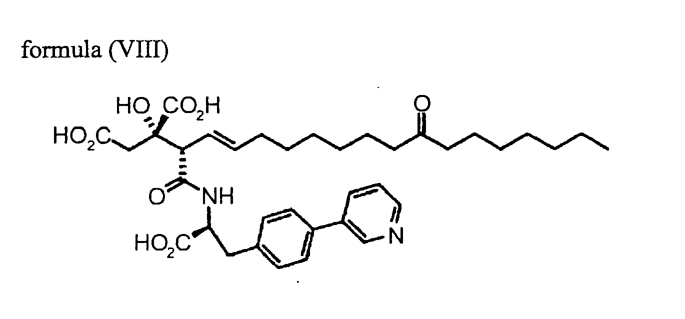

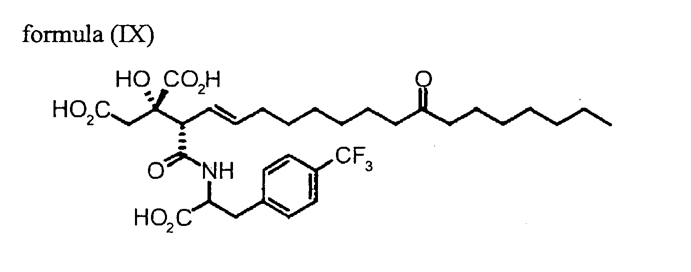

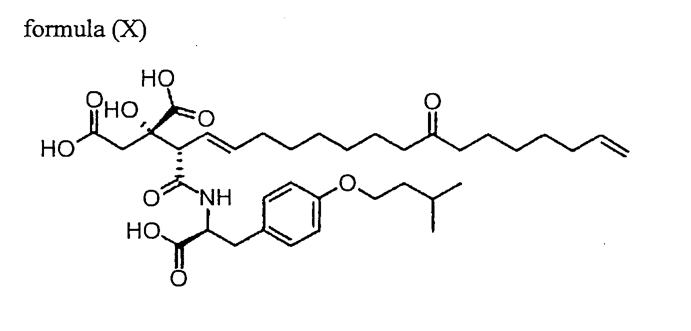

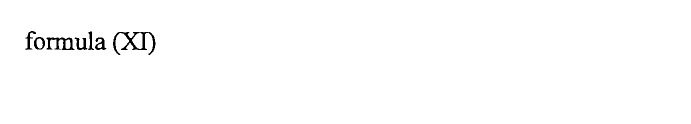

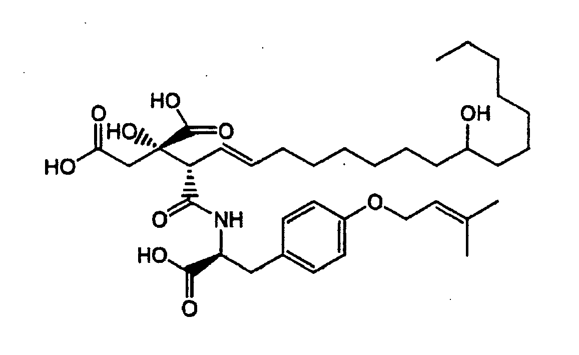

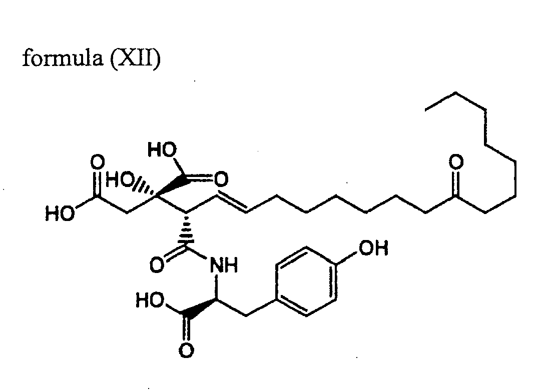

The compounds of the present invention that suppress the expression of serine palmitoyltransferase include RNA complementary to a transcript of DNA encoding serine palmitoyltransferase, and ribozymes that specifically cleave the transcript. In the present invention, the origin of the serine palmitoyltransferase whose expression is to be suppressed is not particularly limited; however, it is preferably derived from a mammal, more preferably from human. The DNA encoding serine palmitoyltransferase includes DNA comprising the nucleotide sequence of SEQ ID NO: 3 or 5 (LCB1 or LCB2); DNA encoding a protein comprising the amino acid sequence of SEQ ID NO: 4 or 6, and naturally-derived DNA encoding a protein comprising an amino acid sequence with a substitution, deletion, addition, and/or insertion of one or more amino acids in the amino acid sequence of SEQ ID NO: 4 or 6. The "protein comprising an amino acid sequence with a substitution, deletion, addition, and/or insertion of one or more amino acids" is functionally equivalent to and shares high homology with a natural protein. The high homology refers to a sequence identity of at least 50% or higher, more preferably 70% or higher, and still more preferably 90% or higher (for example, 95%, 96%, 97%, 98%, 99%, or higher) in the entire amino acid sequence.

The naturally-derived DNA described above hybridizes to DNA comprising the nucleotide sequence of SEQ ID NO: 3 or 5. Hybridization conditions may be appropriately chosen by one skilled in the art. For example, such conditions are 5x SSC, 0.1% SDS at 42°C, or preferably 5x SSC, 0.1% SDS at 50°C, in post-hybridization washes. More preferred hybridization conditions are, for example, 0.1x SSC, 0.1% SDS at 65°C.

In the present invention, the phrase "suppress expression of an enzyme" includes suppressing gene transcription as well as suppressing translation to a protein. The phrase includes not only complete arrest of DNA expression, but also reduced DNA expression.

The RNAs complementary to the transcripts of DNAs encoding serine palmitoyltransferase include more preferably siRNAs set forth in SEQ ID NOs: 1 and 2.

One embodiment of the "RNA complementary to the transcript of a DNA encoding an enzyme" of the present invention is an antisense RNA complementary to the transcript of the DNA encoding the enzyme.

Antisense nucleic acids have multiple factors such as the following to suppress target gene expression. Specifically, these factors include: inhibition of transcription initiation by triple strand formation; suppression of transcription by hybridization with the site where an RNA polymerase has formed a local open loop structure; suppression of transcription by hybridization with the RNA being synthesized; suppression of splicing by hybridization with the junction between an intron and an exon; suppression of splicing by hybridization with the site of spliceosome formation; suppression of mRNA translocation from the nucleus to the cytoplasm by hybridization with mRNA; suppression of splicing by hybridization with the capping site or with the poly A addition site; suppression of translation initiation by hybridization with the binding site for the translation initiation factors; suppression of translation by hybridization with the site for ribosome binding near the initiation codon; inhibition of peptide chain elongation by hybridization with the coding region or with the polysome binding sites of mRNA; and suppression of gene expression by hybridization with the site of interaction between nucleic acids and proteins. These factors suppress target gene expression by inhibiting the process of transcription, splicing, or translation.

Antisense sequences used in the present invention can suppress target gene expression by any of the above factors. In one embodiment, if an antisense sequence is designed to be complementary to a noncoding region near the 5' end of the gene's mRNA, it will effectively inhibit translation of that gene. Sequences complementary to a coding region or to a noncoding region on the 3' side can also be used. Thus, the antisense DNAs used in the present invention also include DNAs comprising antisense sequences against the sequences of both the noncoding and the coding regions of the gene. The antisense DNAs to be used are linked downstream of an appropriate promoter, and preferably, a sequence comprising the transcription termination signal is linked on the 3' side. DNAs thus prepared can be used to transform a desired plant by known methods. The sequence of an antisense DNA is preferably a sequence complementary to an endogenous gene of the plant to be transformed or a part thereof; however, it need not be perfectly complementary, so long as it can effectively inhibit gene expression. The transcribed RNAs are preferably 90% or more, and most preferably 95% or more complementary to transcripts of the target gene. In order to effectively inhibit target gene expression using an antisense sequence, an antisense DNA should be at least 15 nucleotides or more, more preferably at least 100 nucleotides or more, and still more preferably at least 500 nucleotides or more. The antisense DNAs to be used are generally shorter than 5 kb, and preferably shorter than 2.5 kb.

One embodiment of "an RNA complementary to the transcript of a DNA encoding an enzyme" is a dsRNA complementary to the transcript of the DNA encoding the enzyme, and includes the RNAi technique. RNAi is a phenomenon where, when a double-stranded RNA (hereinafter, dsRNA) comprising a sequence identical or similar to a target gene sequence is introduced into cells, the expression of both the introduced foreign gene and the endogenous target gene is suppressed. When approximately 40 to several hundred base pairs of dsRNA are introduced into cells, an RNaseIII-like nuclease called Dicer, which has a helicase domain, excises the dsRNA in the presence of ATP from the 3' end, approximately 21 to 23 base pairs at a time, and produces short interference RNAs (siRNAs). Binding of a specific protein to these siRNAs forms nuclease complexes (RNA-induced silencing complex: RISC). These complexes recognize and bind to sequences the same as those of the siRNAs, and cleave the mRNAs of the target gene in the middle of the siRNAs using RNaseIII-like enzyme activity. In addition to this pathway, the antisense siRNA strands bind to mRNAs and act as primers for RNA-dependent RNA polymerase (RdRP) to synthesize dsRNAs. Pathways in which these dsRNAs again become Dicer substrates and produce new siRNAs to amplify their action can also be considered.

The RNAs of the present invention can be expressed from antisense coding DNAs that encode antisense RNAs for any region of a target gene mRNA, and from sense coding DNAs that encode sense RNAs for any region of a target gene mRNA. dsRNAs can also be produced from such antisense and sense RNAs.

When a dsRNA expression system of the present invention is incorporated into a vector or the like, the antisense and sense RNAs may be expressed from the same vector, or they may be expressed from different vectors. For example, to express an antisense RNA and a sense RNA from the same vector, an antisense RNA expression cassette and a sense RNA expression cassette in which a promoter that may initiate expression of a short RNA, such as the pol III system, is linked upstream of each of the antisense-encoding DNA and sense-encoding DNA, can be individually constructed; and then these cassettes can be inserted into a vector in the same or opposite direction. Furthermore, an expression system that has the antisense-encoding DNA and sense-encoding DNA positioned in opposite directions so that they face each other on different strands can be composed. In this system, a single double-stranded DNA (siRNA-encoding DNA) in which the antisense RNA-encoding strand and sense RNA-encoding strand are paired is provided, and promoters can be placed on both sides in opposite directions so that the antisense RNA and the sense RNA can be expressed from each of the strands. In this case, to avoid addition of an unnecessary sequence downstream of the sense RNA or antisense RNA, a terminator is preferably placed at the 3' end of each of the strands (the antisense RNA-encoding strand and the sense RNA-encoding strand). A sequence of four or more continuous adenine (A) nucleotides can be used as this terminator. Furthermore, in this palindromic expression system, the two promoters are preferably different types.

To express antisense and sense RNAs from different vectors, for example, an antisense RNA expression cassette and a sense RNA expression cassette in which a promoter that may initiate expression of a short RNA, such as the pol III system, is linked upstream of each of the antisense-encoding DNA and the sense-encoding DNA, can be individually constructed; and then these cassettes can be incorporated into different vectors.

In RNAi of the present invention, siRNAs may be used as the dsRNAs. The term "siRNA" refers to a double-stranded RNA comprising short chains in a range that does not show toxicity within cells, for example, 15 to 49 base pairs long, preferably 15 to 35 base pairs long, and more preferably 21 to 30 base pairs long. Alternatively, the siRNAs to be expressed may be transcribed such that the final length of the double-stranded RNA portion may be, for example, 15 to 49 base pairs, preferably 15 to 35 base pairs, and more preferably 21 to 30 base pairs.

The DNAs used for RNAi need not be completely identical to the target gene, but should have a sequence identity of at least 70% or more, preferably 80% or more, more preferably 90% or more, and most preferably 95% or more.

In dsRNAs, the double-stranded portion in which the RNAs are paired is not limited to portions that are completely paired, and may comprise unpaired portions caused by mismatches (in which the corresponding nucleotides are not complementary), bulges (in which one of the strands lacks corresponding nucleotides), and such. In the present invention, the double-stranded RNA regions, in which the RNAs of dsRNAs are paired, may comprise both bulges and mismatches.

DNAs that encode ribozymes can also be used to "suppress expression of an enzyme" in the present invention. A ribozyme is an RNA molecule with catalytic activity. Some ribozymes have various activities, and among them, research on ribozymes that work as RNA-cleaving enzymes has enabled the design of ribozymes aimed at site-specific cleavage of RNAs. Some ribozymes such as those of group I intron type or the M1RNA contained in RNaseP consist of 400 nucleotides or more, while others belonging to the hammerhead type or the hairpin type have an active domain of about 40 nucleotides.

For example, the self-cleavage domain of a hammerhead type ribozyme cleaves at the 3' side of C 15 of the sequence G13U14C15, but formation of a base pair between U14 and A at the ninth position is considered important for the ribozyme activity, and it has been shown that the cleavage also occurs when the nucleotide at the 15th position is A or U instead of C. If the substrate binding site of the ribozyme is designed to be complementary to an RNA sequence adjacent to the target site, a restriction-enzyme-like RNA cleaving ribozyme which recognizes the sequence UC, UU, or UA within the target RNA can be created. For example, multiple sites that can be used as the ribozyme target are present in the coding region of an enzyme of the present invention that will be the target of inhibition.

A hairpin type ribozyme is also useful for an objective of the present invention. Hairpin type ribozymes can be found, for example, in the minus strand of the satellite RNAs of Tobacco ringspot virus (J. M. Buzayan, Nature 323: 349 (1986)). It has been shown that this ribozyme can also be designed to target-specifically cleave RNA.

The ribozyme designed to cleave the target is linked to a transcription termination sequence and to a promoter such as Cauliflower mosaic virus 35S promoter so that it will be transcribed in plant cells. However, if extra sequences have been added to the 5' end or 3' end of the transcribed RNA, the ribozyme activity can be lost. In this case, one can place an additional trimming ribozyme, which functions in *cis* to perform the trimming on the 5' or the 3' side of the ribozyme portion, in order to precisely cut the ribozyme portion alone from the transcribed RNA containing the ribozyme (K. Taira et al. (1990) Protein Eng. 3: 733; A. M. Dzaianott and J. J. Bujarski (1989) Proc. Natl. Acad. Sci. USA 86: 4823; C. A. Grosshands and R. T. Cech (1991) Nucleic Acids Res. 19: 3875; K. Taira et al. (1991) Nucleic Acid Res. 19: 5125). Multiple sites within the target gene can be cleaved by arranging these structural units in tandem to achieve greater effects (N. Yuyama et al., Biochem. Biophys. Res. Commun. 186: 1271 (1992)). By using such ribozymes, it is possible to specifically cleave the transcripts of the target gene of the present invention to suppress its gene expression.

In the present invention, the "interferon" collectively refers to proteins or glycoproteins that have an antiviral action and are induced from animal cells by viruses, double stranded RNA, lectin, and such. In addition to the antiviral action, interferons have a cell growth-suppressing action and an immunoregulatory action. They are categorized into several types according to the cells producing them, the binding ability to specific receptors, and biological and physicochemical characteristics. The major types are α, β, and γ, and other types that are known to exist are IFNω, and IFNτ. Furthermore, 20 or more subtypes of interferon α are known to exist. At present, not only naturally-derived formulations but also various genetically recombinant type formulations, such as PEG-interferons and consensus interferons have been developed and are commercially available.

The interferons of the present invention may be of any type described above; however, interferons α and γ are preferred. Furthermore, the interferons of the present invention may be a natural type, genetic recombinant type which is artificially mutated, naturally-occurring mutant, fusion protein, a fragment thereof, or such, as long as it enhances the ability to suppress the HCV proliferation when used in combination with a compound that blocks a process of sphingomyelin biosynthesis. Furthermore, the interferons of the present invention may be PEG(polyethylene glycol)ylated. Interferons can be pegylated by methods known to those skilled in the art (Japanese Patent No. 2980569).

The interferons according to the present invention are not particularly limited in terms of their origin. For example, the interferons can be derived from humans, chimpanzees, orangutans, dogs, horses, sheep, goats, donkeys, pigs, cats, mice, guinea pigs, rats, rabbits, or such; however, the origin is not limited thereto, and the interferons can also be derived from other mammals. Preferably, the interferons are derived from humans.

The amino acid sequences of human interferons α and γ are known. For example, the amino acid sequence of GenBank: NM_0240013 can be used for interferon α, and the amino acid sequence of GenBank: NM_000619 can be used for interferon γ. The amino acid sequence and nucleotide sequence of interferon α are shown in SEQ ID NOs: 7 and 8 respectively, and the amino acid sequence and nucleotide sequence of interferon γ are shown in SEQ ID NOs: 9 and 10 respectively. The above-mentioned interferons can be prepared by methods well known to those skilled in the art. For example, they can be prepared by preparing mRNAs from interferon-producing cells derived from humans by a generally known technique to prepare a cDNA library; selecting from the cDNA library, cDNAs that hybridize under stringent conditions with a probe comprising all or a part of the nucleotide sequence of SEQ ID NO: 8 or 10; expressing the cDNAs using a suitable host-vector system; and purifying the obtained proteins. A host-vector system selected from the later-described examples of host-vector systems that are applicable to antibody production may be used. Alternatively, the interferons can be prepared by designing primers based on the nucleotide sequences of SEQ ID NO: 8 or 10, performing RT-PCR using mRNAs prepared from human-derived interferon producing cells as templates and using the above-mentioned primers, and then expressing the obtained cDNAs.

Those skilled in the art can appropriately select the above stringent hybridization conditions. For example, pre-hybridization is carried out in a hybridization solution containing 25% formamide, or 50% formamide under more stringent conditions, and 4x SSC, 50 mM Hepes (pH7.0), 10x Denhardt's solution, and 20 µg/ml denatured salmon sperm DNA at 42°C overnight. A labeled probe is then added to the solution and hybridization is carried out by incubation at 42°C overnight. Post-hybridization washes can be carried out with conditions for washing solution and temperature of about "1x SSC, 0.1% SDS, 37°C", or more stringent conditions of about "0.5x SSC, 0.1% SDS, 42°C", or more highly stringent conditions of about "0.2x SSC, 0.1% SDS, 65°C". As the stringency of the post-hybridization washes increases, polynucleotides with greater homology to the probe sequence are expected to be isolated. The above-described combinations of SSC, SDS, and temperature are merely examples of washing conditions. Those skilled in the art can achieve the same astringencies as those described above by appropriately combining the above factors or others (such as probe concentration, probe length, or hybridization period) that affect hybridization stringency.

Polypeptides encoded by polynucleotides isolated using such hybridization techniques will usually comprise amino acid sequences highly homologous to the polypeptides identified by the present inventors. "High homology" refers to sequence homology of at least 40% or more, preferably 60% or more, further preferably 80% or more, further preferably 90% or more, further preferably at least 95% or more, and further preferably at least 97% or more (for example, 98% to 99%). Amino acid sequence identity can be determined, for example, using the BLAST algorithm of Karlin and Altschul (Proc. Natl. Acad. Sci. USA. 87:2264-2268, 1990; Proc. Natl. Acad. Sci. USA 90: 5873-5877, 1993). A program called BLASTX has been developed based on this algorithm (Altschul et al., J. Mol. Biol. 215: 403-410, 1990). When using BLASTX to analyze amino acid sequence identity, the parameters are, for example, score = 50 and wordlength = 3. When using the BLAST and Gapped BLAST programs, the default parameters for each program are used. Specific methodology for these analysis methods is well known (http://www.ncbi.nlm.nih.gov).

Furthermore, without limitation to the interferons of the above-mentioned sequences, polypeptides similar to the interferons of the above-mentioned sequences can also be suitably used in the present invention so long as they enhance the ability to suppress HCV proliferation when used in combination with a compound that blocks a process of sphingomyelin biosynthesis. Examples of such polypeptides include a polypeptide that has a high ability to suppress HCV proliferation when used in combination with a compound that blocks a process of sphingomyelin biosynthesis and comprises an amino acid sequence with one or more amino acid deletions, substitutions, additions, and/or insertions in the amino acid sequence of SEQ ID NO: 7; a polypeptide that has a high ability to suppress HCV proliferation when used in combination with a compound that blocks a process of sphingomyelin biosynthesis and comprises an amino acid sequence with one or more amino acid deletions, substitutions, additions, and/or insertions in the amino acid sequence of SEQ ID NO: 9; a polypeptide that has a high ability to suppress HCV proliferation when used in combination with a compound that blocks a process of sphingomyelin biosynthesis and comprises an amino acid sequence encoded by a nucleotide sequence that hybridizes under stringent conditions with the nucleotide sequence of SEQ ID NO: 8; and a polypeptide that has a high ability to suppress HCV proliferation when used in combination with a compound that blocks a process of sphingomyelin biosynthesis and comprises an amino acid sequence encoded by a nucleotide sequence that hybridizes under stringent conditions with the nucleotide sequence of SEQ ID NO: 10.

Such polypeptides can be prepared by methods well known to those skilled in the art. For example, all or a portion of the nucleotide sequence of SEQ ID NO: 8 or 10 can be used as a probe to select hybridizing clones from a cDNA library prepared from interferon-producing cells, and the clones can be expressed to prepare the polypeptides. Alternatively, the polypeptides can be prepared by performing gene modification methods well known to those skilled in the art, such as PCR mutagenesis or cassette mutagenesis, on the nucleotide sequence of SEQ ID NO: 8 or 10 to site-specifically or randomly introduce mutations. It is also possible to synthesize sequences with mutations which have been introduced into the nucleotide sequence of SEQ ID NO: 8 or 10, by using a commercially available nucleic acid synthesizer.

Herein, the term "treatment" means that HCV is eliminated or reduced, further spread of HCV is suppressed, and symptoms of HCV infection are alleviated, by administering agents of the present invention to the subjects. Furthermore, the term "prevention" means that HCV infection is prevented or HCV proliferation is suppressed by administering agents of the present invention to the subjects before HCV infection. Symptoms of HCV infection include type C hepatitis, cirrhosis, hepatic fibrosis, and hepatic cancer.

In the present invention, the combined use of an interferon and a compound that blocks a process of sphingomyelin biosynthesis means that both interferon and compound that blocks a process of sphingomyelin biosynthesis are administered or used (hereinafter simply referred to as "administration"), but it is not limited by the order or interval of administration. It is also possible to combine an interferon and a compound of the present invention as a kit. Furthermore, when an interferon and a compound of the present invention are used in combination, if desired, each dose can also be reduced as compared to when either is used alone.

The compound of the present invention and interferon may be administered in any order: interferon may be administered after administering the compound; the compound and interferon may be administered simultaneously; or the compound may be administered after administering interferon.

When the compound of the present invention and interferon are administered individually, the interval between the administration of the compound and interferon is not particularly limited and can be determined in consideration of factors such as administration route and dosage form. A specific example of the administration interval is typically 0 to 168 hours, preferably 0 to 72 hours, more preferably 0 to 24 hours, and still more preferably 0 to 12 hours.

Furthermore, the administration interval of interferon is typically once a day to once a month, preferably once a week, but is not limited thereto. Furthermore, the administration interval of a compound that blocks a process of sphingomyelin biosynthesis is typically once a day to once every two months, preferably once a day to once a month, but is not limited thereto.

When an interferon and the compound of the present invention are administered separately, their administration method and number of daily doses may be the same or different. Furthermore, the weight ratio of interferon and the compound of the present invention is also not particularly limited.

The compounds of the present invention can be used as pharmaceutical agents directly or in the form of pharmaceutically acceptable salts. The above-mentioned salts are not particularly limited, so long as they are pharmaceutically acceptable, and examples include salts formed with mineral acids such as hydrochloric acid, sulfuric acid, nitric acid, phosphoric acid, and hydrobromic acid; salts formed with organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid; and salts formed with alkali metals or alkaline earth metals such as sodium, potassium, and calcium.

The amount of an active ingredient compound comprised in the above-mentioned pharmaceutical preparation is not particularly limited and can be appropriately selected in a wide range; however, examples are 0.1% to 99.5% by weight, or preferably 0.5% to 90% by weight.

A compound of the present invention can be formulated as the base according to conventional methods using known adjuvants that may be used ordinarily in the art of pharmaceutical preparation, such as excipients, binders, disintegrators, lubricants, flavoring agents, solubilizing adjuvants, suspending agents, and coating agents. When shaping into the form of tablets, a wide variety of substances conventionally known as carriers in the art can be used, and examples include excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrators such as dried starch, sodium alginate, agar powder, laminaran powder, sodium bicarbonate, calcium carbonate, polyoxyethylene sorbitan fatty acid esters, sodium lauryl sulfate, stearic acid monoglyceride, starch, and lactose; disintegration inhibitors such as sucrose, stearic acid, cacao butter, and hydrogenated oil; absorbefacients such as quaternary ammonium salts and sodium lauryl sulfate; moisturizers such as glycerin and starch; adsorbents such as starch, lactose, kaolin, bentonite, and colloidal silicic acid; and lubricants such as purified talc, stearate, boric acid powder, and polyethylene glycol.

Tablets can be prepared, as necessary, as ordinary coated tablets, such as sugar-coated tablets, gelatin-coated tablets, enteric-coated tablets, and film-coated tablets, or as double layered tablets or multilayered tablets. When shaping into the form of pills, a wide variety of substances conventionally known as carriers in the art can be used, and examples include excipients such as glucose, lactose, cacao butter, starch, hardened vegetable oil, kaolin, and talc; binders such as gum arabic powder, tragacanth powder, gelatin, and ethanol; and disintegrators such as laminaran agar. When shaping into the form of suppositories, a wide variety of substances conventionally known as carriers in this field can be used, and examples include polyethylene glycol, cacao butter, higher alcohols, esters of higher alcohols, gelatin, and semi-synthetic glycerides. When preparing injections, solutions and suspensions are sterilized and are preferably isotonic with blood, and when making these solution, emulsion, and suspension forms, any substances commonly used as diluents in the field can be used, such as water, ethanol, propylene glycol, ethoxylated isostearyl alcohol, polyoxylated isostearyl alcohol, and polyoxyethylene sorbitan fatty acid esters. In these instances, adequate amounts of sodium chloride, glucose, or glycerin can be comprised in the pharmaceutical preparations to prepare isotonic solutions, and ordinary solubilizing adjuvants, buffers, analgesic agents, and such may also be added. The pharmaceutical preparations may further comprise, as necessary, coloring agents, preservatives, flavors, flavoring agents, and sweeteners, as well as other pharmaceutical agents.

The above-mentioned pharmaceutical compositions are preferably administered in unit dosage forms, and can be administered orally, interstitially (subcutaneously, intramuscularly, intravenously, and such), locally (percutaneously), or transrectally. The pharmaceutical compositions are obviously administered in dosage forms suited to these administration methods.

When administering the compounds of the present invention or pharmaceutically acceptable salts thereof as pharmaceutical agents, the doses of the antiviral agents are preferably adjusted after considering the patient's conditions such as age and weight, the administration route, and the property and degree of the disease; however, for humans, the daily dose of the active ingredient of the present invention for adults is ordinarily within the range of 0.1 mg to 2000 mg. While doses lower than the above-mentioned range may be sufficient in some cases, doses higher than this range may be required in other cases. When a high dose is used, the daily dosage is preferably administered in several divided doses.

The above-mentioned oral administration can be carried out using solid, powdered, or liquid dosage units, such as powders, powdered drugs, tablets, sugar-coated agents, capsules, drops, sublingual tablets, and other dosage forms.

The above-mentioned interstitial administration can be carried out, for example, using liquid unit dosage forms for subcutaneous, intramuscular, or intravenous injections, such as solutions and suspensions. These are produced by suspending or dissolving a certain amount of a compound of the present invention or a pharmaceutically acceptable salt thereof, in a non-toxic liquid carrier suitable for purposes of injection, such as an aqueous or oily medium, and then sterilizing this suspension or solution.

The above-mentioned local administration (percutaneous administration and such) can be carried out using external preparation forms such as solutions, creams, powders, pastes, gels, and ointments. These are produced by combining a certain amount of a compound of the present invention or a pharmaceutically acceptable salt thereof, with one or more of a flavor, coloring agent, filler, surfactant, moisturizer, emollient, gelling agent, carrier, preservative, and stabilizer suited to the aim of the external preparation.

The above-mentioned transrectal administration can be carried out using suppositories and the like, prepared by mixing a certain amount of a compound of the present invention or a pharmaceutically acceptable salt thereof into a low-melting solid comprising, for example, higher esters such as myristyl palmitate ester, polyethylene glycol, cacao butter, or a mixture thereof.

The above-mentioned administrations can be carried out using liquid unit dosage forms for subcutaneous, intramuscular, or intravenous injections, such as solutions or suspensions. They are produced by suspending or dissolving a certain amount of a compound of the present invention or a pharmaceutically acceptable salt thereof, in a non-toxic liquid carrier appropriate to the purpose of the injection, such as an aqueous or oily medium, and then sterilizing this suspension or solution.

### [Examples]

Herein below, the present invention will be specifically described with reference to the Examples, but it is not to be construed as being limited thereto.

### [Example 1] Anti-HCV activity of the SPT inhibitor myriocin

The present inventors investigated the anti-HCV activity and cytotoxicity of myriocin in FLR3-1 HCV subgenomic replicon cells.

First, myriocin (Sigma, St. Louis, MO, USA) was added at a final concentration of 0.2, 1.0, 3.9, 15.6, or 62.5 nM to a growth medium for FLR3-1 HCV subgenomic replicon cells (genotype 1b; Con-1; Sakamoto, H. et al., Nat Chem Biol, 1, 333-337 (2005)). After 72 hours of incubation, the present inventors carried out luciferase assay using the Bright-Glo luciferase assay kit (Promega, Madison, WI, USA).

Furthermore, myriocin was added to FLR3-1 cells, and after 72 hours of incubation, the cell viability was determined using Tetra Color One kit (Seikagakukogyo, Tokyo, Japan) according to the manufacturer's instructions.

The result showed that myriocin significantly reduced the luciferase activity in a dose-dependent manner without affecting the cell viability (Fig. 2A) or cell growth (data not shown). The maximum inhibitory rate in the presence of 62.5 nM myriocin was about 80% (Fig.2A), while the 50% inhibitory concentration (IC₅₀) was about 5.8 nM (Table 1).

**Table 1: IC₅₀ of myriocin**

| Agent added (µM) | | IC₅₀ of myriocin (nM) |
|---|---|---|
| No agent added | 0 | 5.8 |
| Dihydrosphingosine | 1.0 | 77.7 |
| | 2.5 | > 1000 |
| Sphingosine | 1.0 | 22.4 |
| | 2.5 | > 1000 |
| Sphingosine-1-phosphate | 1.0 | 14.7 |
| | 2.5 | > 1000 |

Immunoblotting analysis and immunofluorescent staining showed that NS3 protein, which plays an important role in HCV replication, was also reduced. This suggests that myriocin has a strong anti-HCV effect (Fig. 3).

### [Example 2] Correlation between sphingolipid metabolites and HCV replication

To investigate the correlation between sphingolipid metabolites and HCV replication, the present inventors monitored the *de novo* sphingolipid biosynthesis in FLR3-1 cells in the presence of myriocin.

First, FLR3-1 cells were incubated with [¹⁴C]-serine (0.5 µCi/ml) in Opti-MEM (Invitrogen) for two hours. After the cells were lysed in 0.1% SDS, total lipid was extracted using chloroform/methanol (1:2 (v/v)). The extract was spotted onto a Silica gel 60 thin layer chromatography (TLC) plate (Merck, Darmstadt, Germany), and chromatographed using methyl acetate/ 1-propanol/ chloroform/methanol/ 0.25% KCl (25:25:25:10:9 (v/v)). Radioactive spots were detected using BAS 2000 (Fujifilm, Kanagawa, Japan).

The result demonstrated that the production of both ceramide and sphingomyelin was inhibited in a dose dependent manner, while the production of phosphatidylethanolamine and phosphatidylserine, which are metabolites of sphingosine, was not affected (Fig. 2B).

To confirm whether the suppression of the replication of HCV subgenomic replicon results from sphingolipid depletion, the present inventors examined the anti-HCV effect of myriocin in the presence of an intermediate in the sphingolipid biosynthetic pathway, *i.e.* dihydrosphingosine, sphingosine, or sphingosine-1-phosphate (Fig. 1).

FLR3-1 cells were incubated with 1 or 2.5 µM of a sphingolipid intermediate (dihydrosphingosine, sphingosine, or sphingosine-1-phosphate), and then serial dilutions of myriocin were added thereto. After 72 hours, IC₅₀ in each combination was determined by luciferase assay.

It was revealed that the replication ability of HCV replicon was restored by supplementing an intermediate molecule of the sphingolipid biosynthesis (Table 1). This result shows that the suppression of the replication by myriocin is attributed to the suppression of sphingolipid biosynthesis.

### [Example 3] Anti-HCV effect of myriocin and PEG-IFN in chimeric mice infected with HCV

The inhibitory activity of myriocin was examined using chimeric mice having humanized liver infected with HCR6 (genotype 1b). The chimeric mice used were purchased from PhoenixBio Co. (Hiroshima, Japan).

Specifically, according to Table 2, myriocin and/or PEG-IFN (Chugai, Tokyo, Japan) were administered intraperitoneally or subcutaneously into mice infected with HCV genotype 1b (HCR6; accession number AY045702) and blood was collected.

**Table 2: Administration schedule for chimeric mice infected with HCV genotype 1b**

| Day | -1 | 0 | 1 | 2 | 3 | 4 | 5 | 6 | 7 | 8 |
|---|---|---|---|---|---|---|---|---|---|---|
| Blood collection | B | | B | | | B | | | | B |
| PEG - IFN | | I | | | I | | | | I | |
| Myriocin | | M | M | M | M | M | M_{1/2} | M_{1/2} | M_{1/2} | |
| Myriocin + PEG - IFN | | M/I | M | M | M/I | M_{1/2} | M_{1/2} | | I | |

In Table 2, the symbols B, I, and M indicate that each step was conducted as needed and the administration of agents was started on day 0. PEG-IFN was injected at a dose of 30 µg/kg. The dose of myriocin injection was varied depending on the mouse weight. The administration was started at 1 mg/kg (M). When the weight was reduced by 10%, the dose was reduced to 0.5 mg/kg (M_{1/2}). The administration was discontinued when the weight was reduced by 20%.

Next, total RNAs were purified using AGPC method from 1 µl of sera or hepatic tissues collected from the chimeric mice. HCV RNA was quantified by real-time PCR using a method known to those skilled in the art (Takeuchi, T. et al., Gastroenterology, 116, 636-642 (1999)).

The result showed that the serum level of HCV RNA in the myriocin-treated group was reduced from 3 x 10⁶ to 1 x 10⁷ copies/ml to 6 x 10⁵ to 1 x 10⁴ copies/ml (about 10 to 100 times reduction) after eight days. The same level of reduction was seen in the PEG-IFN-treated group in which PEG-IFN was injected at a dose ten times higher than that used in clinical treatment (30 µg/kg weight). Furthermore, the treatment of the combination of myriocin and PEG-IFN reduced the HCV RNA level to less than 1/1000 of the control level, and no HCV RNA was detectable in two mice (1b-7 and 1b-9) of the three on day 8 (Fig. 4A).

The present inventors monitored the human albumin (h-Alb) level at the same time. The human albumin level was determined using 2 µl of serum and the Alb-II kit (Eiken Chemical, Tokyo, Japan) according to the manufacturer's instructions. The result showed that the human albumin level was slightly reduced only in the combination-treated group (Fig. 4B).

These results demonstrate that myriocin suppresses the replication of intact HCV without affecting h-Alb expressed from the humanized liver. They also demonstrate that the combined use of myriocin and PEG-IFN synergistically suppresses the HCV replication while it results in mild liver damage.

### [Example 4] Detection of HCV RNA and core protein in the liver of chimeric mice

To demonstrate whether myriocin reduces HCV in the humanized liver, the present inventors examined the liver of chimeric mice infected with another HCV genotype, 1a (HCG9). The serum RNA level in these mice reached about 1x10⁸ copies/ml, 10 times higher than that of HCV 1b (HCR6) (Fig. 5A). Therefore, it was assumed that the HCV core protein in the hepatocytes could be readily detected by immunofluorescent staining. The present inventors administered 2 mg/kg of myriocin to 1a-4 chimeric mice every day for six days and then excised the livers. For comparison, livers were also excised from non-treated mice (1a-1), non-infected mice (1a-2), and PEG-IFN-treated mice (1a-3) in the same way. The livers were homogenized in RIPA, and 100 µg of the total protein was used for detection of the core protein using the Ortho HCV core protein ELISA kit (Eiken Chemical).

Next, the liver tissues of the chimeric mice were examined by immunofluorescent staining. Liver sections from the 1a-1 and 1a-4 mice were probed using a biotinylated anti-HCV core protein monoclonal antibody and anti-human hepatocyte monoclonal antibody (Dako, Glostrup, Denmark) as primary antibodies. Then, the sections were probed with streptavidin-Alexa 488 (Invitrogen) and anti-mouse IgG-Alexa-546 (Invitrogen). Furthermore, nuclei were stained with DAPI.

The effect of myriocin treatment on the expression of HCV RNA and core protein in the livers of the chimeric mice was confirmed by the method described above.

The result demonstrated that the serum level of HCV RNA in the 1a-4 mice was reduced to 1 x 10⁵ copies/ml after myriocin treatment (Fig. 5A). Furthermore, quantitation of HCV 1a RNA and core protein in the liver showed that they were also reduced as in the serum (Fig. 5A). Immunofluorescent staining revealed that while the core protein was expressed in human hepatocytes of non-treated mice 1a-1 (Fig. 5A), which showed 1x10⁸ HCV copies/ml of serum, the core protein was disappeared in 1a-4 mice (Fig. 5B). These results demonstrate that myriocin reduces not only HCV genotype 1b but also HCV genotype 1a, and eliminates HCV from the liver.

### [Example 5] Anti-HCV effect of the compound of formula (III) and PEG-IFN in chimeric mice infected with HCV

HCR6 (HCV genotype 1b; accession number: AY045702) was administered at 10⁶ copies/mouse to chimeric mice by intravenous injection. After four weeks, the HCV 1b RNA level in the mouse serum reached 10⁶ to 10⁸ copies/ml.

The compound of formula (III) and/or PEG-IFN was administered by the following procedure.

The compound of formula (III) is represented by the following formula:

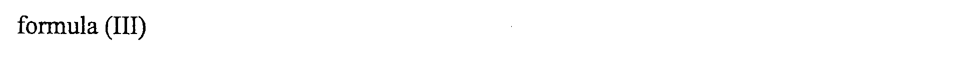

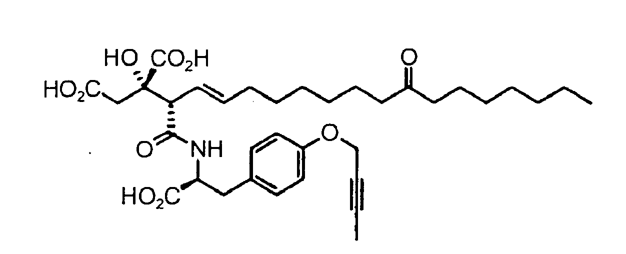

The compound shown above can be synthesized by the methods described in Example 4 of WO 2005/005372.

PEG-IFN (Pegasys; pegylated interferon α-2a) was subcutaneously administered at 3 0 µg/kg twice a week, while the compound of formula (III) was intravenously administered at 5, 10, or 20 mg/kg every day. When the compound of formula (III) and PEG-IFN were used in combination, 10 mg/kg of the compound of formula (III) and 30 µg/kg of PEG-IFN were administered subcutaneously. After administering the compound of formula (III) and/or PEG-IFN to mice infected with HCV genotype 1b, the blood was collected over time.

The anti-HCV activity was assessed by quantifying HCV RNA using real-time PCR. Total RNAs were purified from 1 µl of serum of the chimeric mice, and HCV RNA was quantified by real-time PCR.

Among the 8-day treated groups, the 5 mg/kg-administered group showed a reduction of HCV titer to -1.0 to -1.1 log (mice 1 and 2). In the 10 mg/kg-administered group, the maximal effect was determined to be -1.2 to -1.8 log (mice 3 to 5). The 20 mg/kg-administered group showed a reduction of HCV titer to -1.8 to -2.8 log (mice 6 to 8). Whereas the group administered with PEG-IFN alone (mice 12 to 16) showed a -0.3 to -2 log reduction, the group administered with 10 mg/kg of the compound of formula (III) in combination with PEG-IFN (mice 9 to 11) showed as much as a -2.6 to -4.1 log reduction (Fig. 6). These results demonstrate that the combined use of the compound of formula (III) and PEG-IFN has a synergistic effect in anti-HCV activity. In the 14 day-treated groups, a dose-dependent reduction of the virus was seen with low-dose treatment, but not in the high-dose treatment group.

### Industrial Applicability

The present inventors elucidated the mechanism of inhibition of HCV replication by SPT *inhibitors in vitro* and also demonstrated that SPT inhibitors inhibited the HCV replication in model chimeric mice having humanized liver. The findings obtained by the present inventors indicate that SPT serves as an effective target for agents that are designed to inhibit HCV replication, and SPT inhibitors are lead compounds in developing novel anti-HCV agents.

Furthermore, the combined use of an SPT inhibitor and interferon was demonstrated to synergistically suppress HCV replication. Thus, pharmaceutical compositions containing these two ingredients may be novel, safer, and more effective agents for treating HCV.

## Claims

1. A pharmaceutical composition for treating or preventing HCV infection, which comprises in combination an interferon and a compound that blocks a process of sphingomyelin biosynthesis.

2. The pharmaceutical composition of claim 1, wherein the pharmaceutical composition for treating or preventing HCV infection is a combination agent.

3. The pharmaceutical composition of claim 1, wherein the interferon and the compound that blocks a process of sphingomyelin biosynthesis are used in combination.

4. The pharmaceutical composition of claim 3, wherein the interferon and the compound that blocks a process of sphingomyelin biosynthesis are administered simultaneously or successively.

5. The pharmaceutical composition of claim 3, wherein the interferon and the compound that blocks a process of sphingomyelin biosynthesis are administered separately.

6. A pharmaceutical composition for treating or preventing HCV infection, which comprises as an active ingredient a compound that blocks a process of sphingomyelin biosynthesis, wherein the composition is used in combination with an interferon.

7. A pharmaceutical composition for treating or preventing HCV infection, which comprises an interferon as an active ingredient, wherein the composition is used in combination with a compound that blocks a process of sphingomyelin biosynthesis.

8. The pharmaceutical composition of any one of claims 1 to 7, wherein the compound that blocks a process of sphingomyelin biosynthesis blocks a process of biosynthesis of sphingomyelin from palmitoyl CoA.

9. The pharmaceutical composition of any one of claims 1 to 7, wherein the compound that blocks a process of sphingomyelin biosynthesis is:
(a) a compound that inhibits the enzymatic activity of a serine palmitoyltransferase involved in the biosynthesis of 3-ketodihydrosphingosine from palmitoyl CoA; or
(b) a compound that suppresses the expression of a serine palmitoyltransferase.

10. The pharmaceutical composition of claim 9, wherein the compound that inhibits the enzymatic activity of a serine palmitoyltransferase is myriocin, sphingofungin, a compound represented by formula (1), or a pharmaceutically acceptable salt thereof: 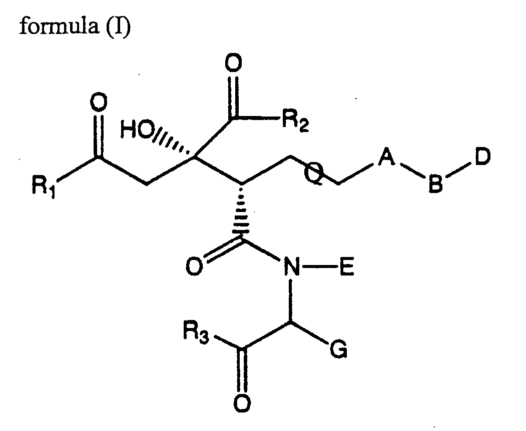 wherein
A represents -(CH₂)ₙ-, wherein n represents an integer from 0 to 10;
B represents -CH₂-, -(C=O)-, -CH(OH)-, -CH(NH₂)-, or -C(=NOR)-, wherein R represents a hydrogen atom, linear or branched alkyl group having 1 to 8 carbon atoms, which is optionally substituted by an amino group which is optionally mono- or di-substituted by a linear or branched alkyl group having 1 to 4 carbon atoms;
D represents -(CH₂)ₘ-R', wherein m represents an integer from 0 to 10, and R' represents a hydrogen atom, linear or branched alkyl group, linear or branched alkynyl group, linear or branched alkenyl group, cycloalkyl group, cycloalkenyl group, optionally substituted heterocyclic group, optionally substituted aryl group, optionally substituted heteroaryl group, -OX group (wherein X represents a hydrogen atom, linear or branched alkyl group, linear or branched alkynyl group, linear or branched alkenyl group, cycloalkyl group, or optionally substituted aryl group), or halogen atom;
E represents a hydrogen atom, or linear or branched alkyl group;
G represents -(CH₂)ₚ-J, wherein p represents an integer from 0 to 4, and J represents a hydrogen, OH group, SH group, methylthio group, carboxyl group, carbamoyl group, amino group, guanidino group, linear or branched alkyl group, cycloalkyl group, linear or branched alkynyl group, linear or branched alkenyl group, optionally substituted aryl group, optionally substituted heterocyclic group, or optionally substituted heteroaryl group;
bond Q represents a single bond or a double bond; and
R¹, R², and R³ are the same or different and each represent a hydroxyl group, amino group (which is optionally mono- or di-substituted by a linear or branched alkyl group having 1 to 4 carbon atoms), -OL, linear or branched alkyl group, linear or branched alkenyl group, or linear or branched alkynyl group, wherein L represents a linear or branched alkyl group, linear or branched alkenyl group, or linear or branched alkynyl group.

11. The pharmaceutical composition of claim 9, wherein the compound that inhibits the enzymatic activity of a serine palmitoyltransferase is a compound represented by any one of formulae (II) to (XII), or a pharmaceutically acceptable salt thereof:

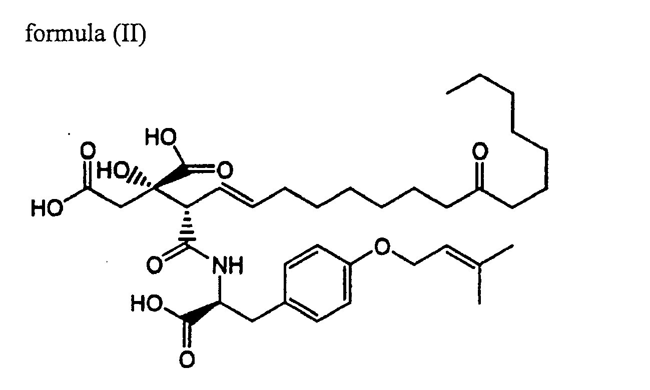

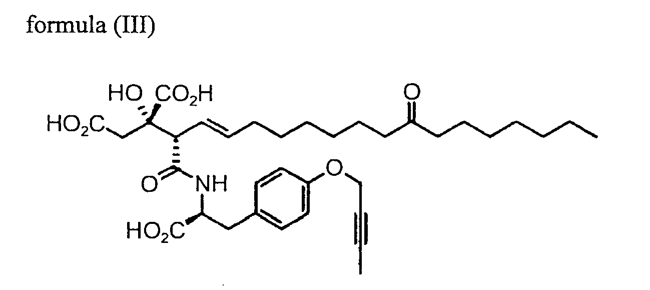

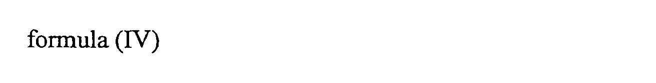

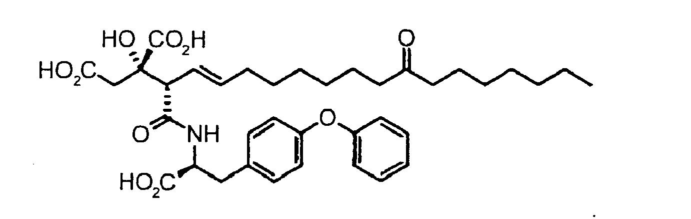

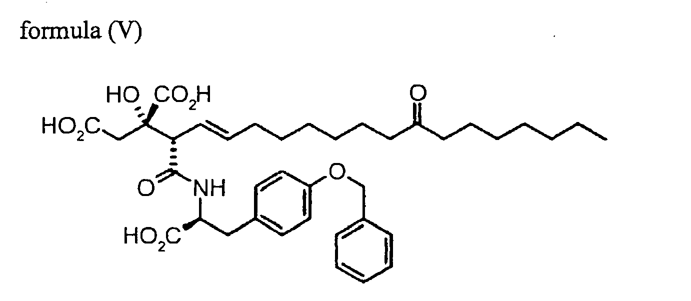

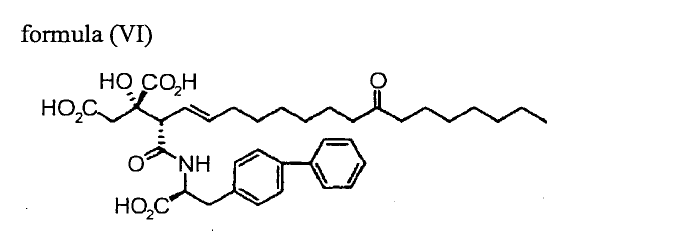

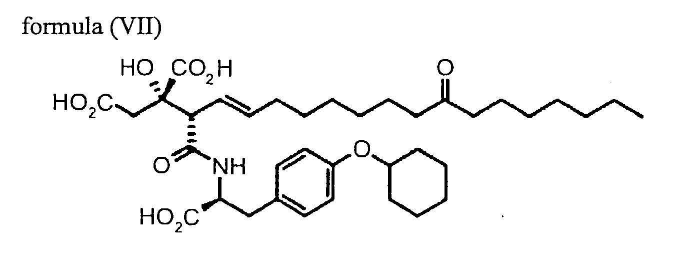

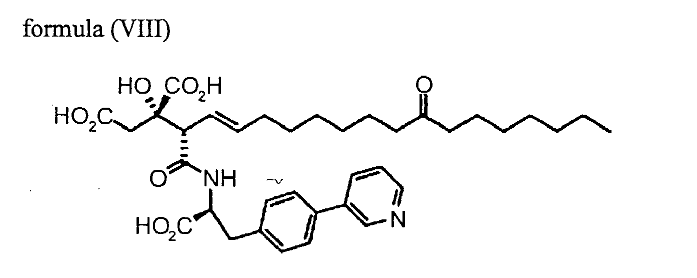

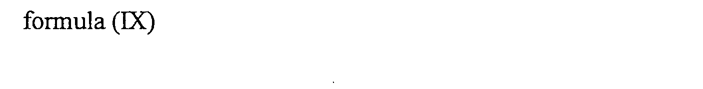

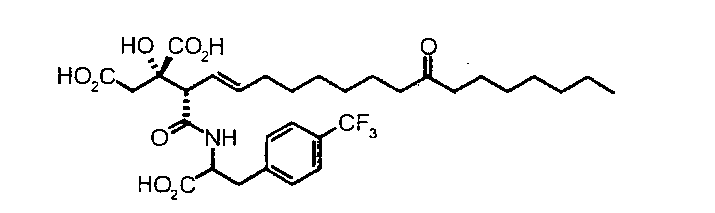

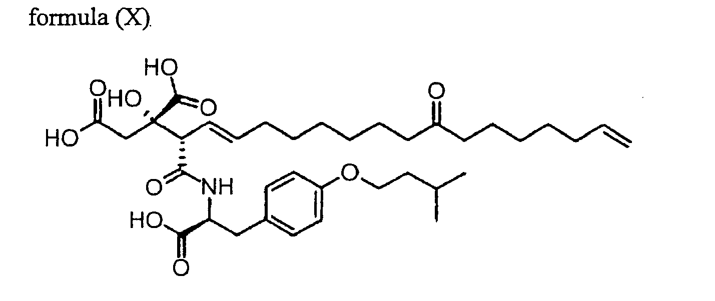

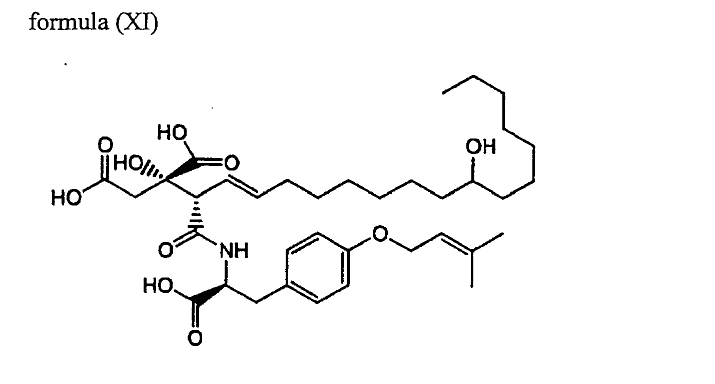

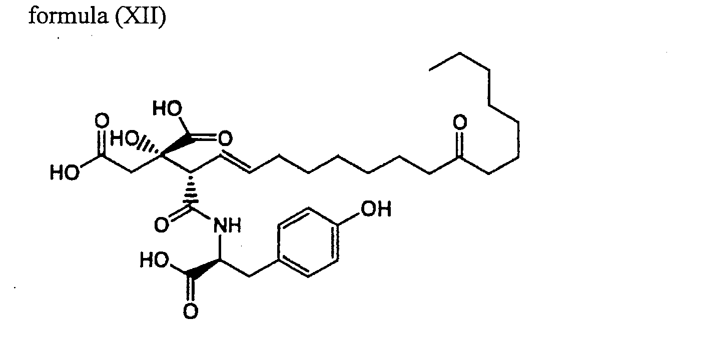

12. The pharmaceutical composition of claim 9, wherein the compound that suppresses the expression of a serine palmitoyltransferase is:
(a) an RNA complementary to a transcript of DNA encoding a serine palmitoyltransferase; or
(b) an RNA having ribozyme activity to specifically cleave a transcript of DNA encoding a serine palmitoyltransferase.

13. The pharmaceutical composition of any one of claims 1 to 7, wherein the interferon is a pegylated interferon.

14. The pharmaceutical composition of any one of claims 1 to 7, wherein the HCV infection is type C hepatitis, cirrhosis, hepatic fibrosis, or hepatic cancer.

15. A method for treating or preventing HCV infection, which comprises the step of administering to a subject an interferon and a compound that blocks a process of sphingomyelin biosynthesis.

16. The method of claim 15, wherein the interferon and the compound that blocks a process of sphingomyelin biosynthesis are simultaneously administered to the subject.

17. The method of claim 15, wherein the interferon and the compound that blocks a process of sphingomyelin biosynthesis are separately administered to the subject.

18. The method of claim 15, wherein the compound that blocks a process of sphingomyelin biosynthesis is a compound that blocks a process of biosynthesis of sphingomyelin from palmitoyl CoA.

19. The method of claim 15, wherein the compound that blocks a process of sphingomyelin biosynthesis is:
(a) a compound that inhibits the enzymatic activity of a serine palmitoyltransferase involved in the biosynthesis of 3-ketodihydrosphingosine from palmitoyl CoA; or
(b) a compound that suppresses the expression of a serine palmitoyltransferase.

20. The method of claim 19, wherein the compound that inhibits the enzymatic activity of a serine palmitoyltransferase is myriocin, sphingofungin, a compound represented by formula (I), or a pharmaceutically acceptable salt thereof: 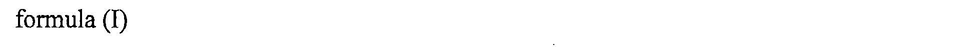 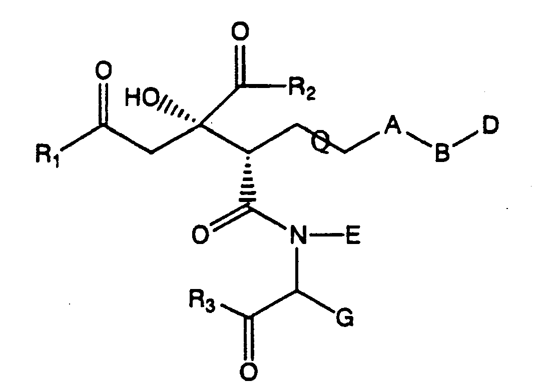 wherein
A represents -(CH₂)ₙ-, wherein n represents an integer from 0 to 10;
B represents -CH₂-, -(C=O)-, -CH(OH)-, -CH(NH₂)-, or -C(=NOR)-, wherein R represents a hydrogen atom, linear or branched alkyl group having 1 to 8 carbon atoms, which is optionally substituted by an amino group which is optionally mono- or di-substituted by a linear or branched alkyl group having 1 to 4 carbon atoms;
D represents -(CH₂)ₘ-R', wherein m represents an integer from 0 to 10, and R' represents a hydrogen atom, linear or branched alkyl group, linear or branched alkynyl group, linear or branched alkenyl group, cycloalkyl group, cycloalkenyl group, optionally substituted heterocyclic group, optionally substituted aryl group, optionally substituted heteroaryl group, -OX group (wherein X represents a hydrogen atom, linear or branched alkyl group, linear or branched alkynyl group, linear or branched alkenyl group, cycloalkyl group, or optionally substituted aryl group), or a halogen atom;
E represents a hydrogen atom, or linear or branched alkyl group;
G represents -(CH₂)ₚ-J, wherein p represents an integer from 0 to 4, and J represents a hydrogen, OH group, SH group, methylthio group, carboxyl group, carbamoyl group, amino group, guanidino group, linear or branched alkyl group, cycloalkyl group, linear or branched alkynyl group, linear or branched alkenyl group, optionally substituted aryl group, optionally substituted heterocyclic group, or optionally substituted heteroaryl group;
bond Q represents a single bond or a double bond; and
R¹, R², and R³ are the same or different and each represent a hydroxyl group, amino group (which is optionally mono- or di-substituted by a linear or branched alkyl group having 1 to 4 carbon atoms), -OL, linear or branched alkyl group, linear or branched alkenyl group, or linear or branched alkynyl group, wherein L represents a linear or branched alkyl group, linear or branched alkenyl group, or linear or branched alkynyl group.

21. The method of claim 19, wherein the compound that inhibits the enzymatic activity of a serine palmitoyltransferase is a compound represented by any one of formulae (II) to (XII), or a pharmaceutically acceptable salt thereof:

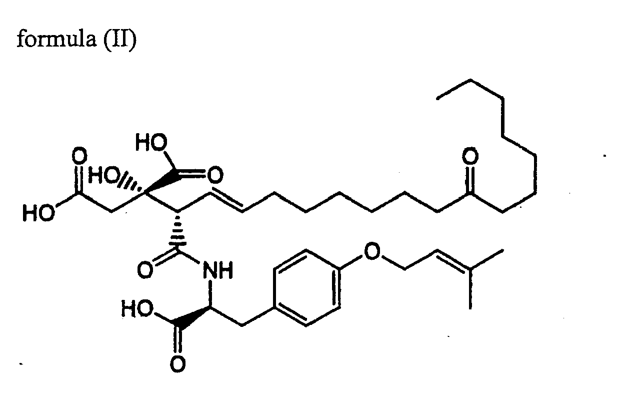

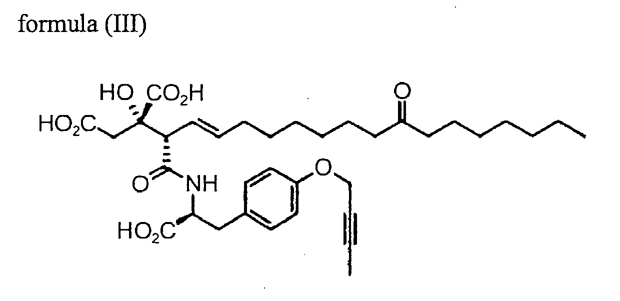

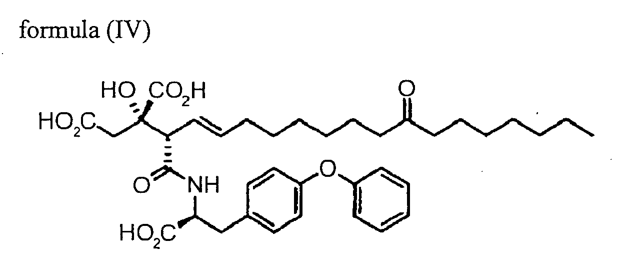

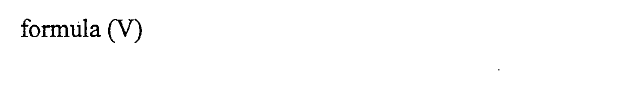

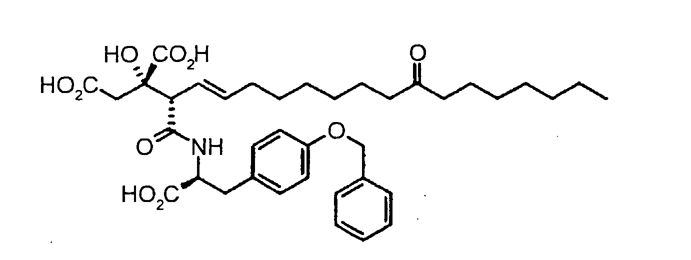

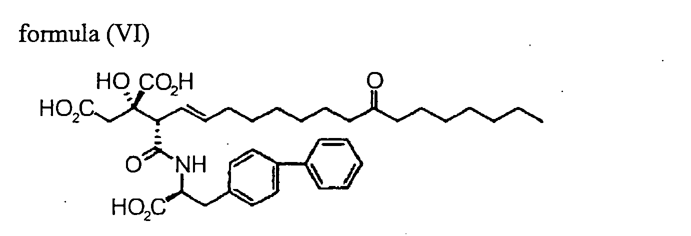

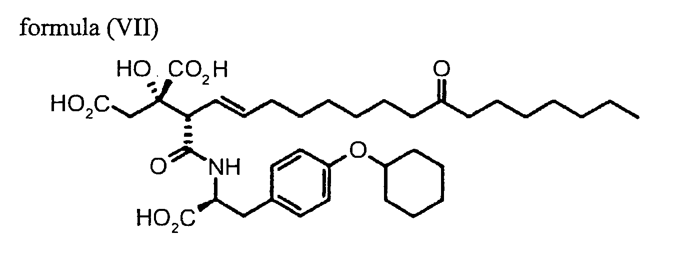

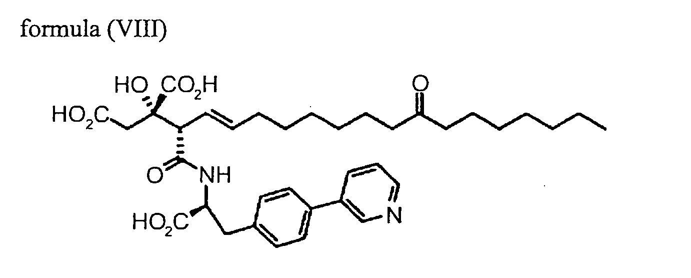

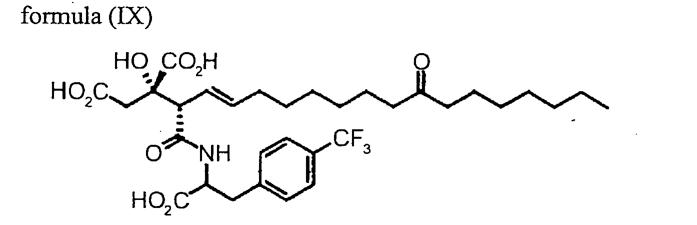

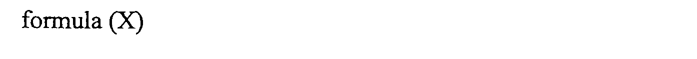

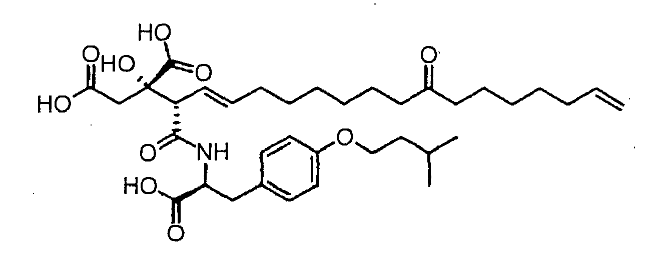

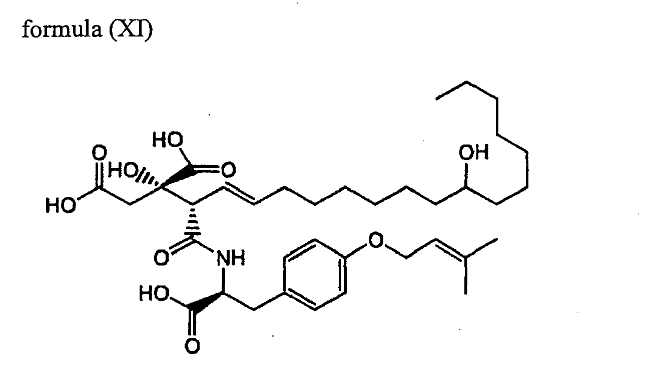

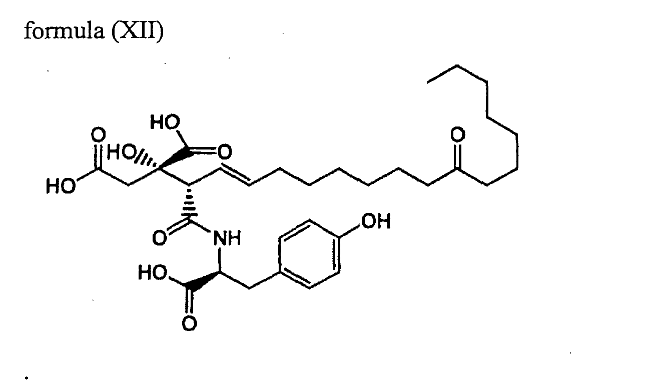

22. The method of claim 19, wherein the compound that suppresses the expression of a serine palmitoyltransferase is:
(a) an RNA complementary to a transcript of a DNA encoding a serine palmitoyltransferase; or
(b) an RNA having ribozyme activity to specifically cleave a transcript of DNA encoding a serine palmitoyltransferase.

23. The method of claim 15, wherein the interferon is a pegylated interferon.

24. The method of claim 15, wherein the HCV infection is type C hepatitis, cirrhosis, hepatic fibrosis, or hepatic cancer.

25. Use of an interferon and a compound that blocks a process of sphingomyelin biosynthesis in the preparation of a pharmaceutical composition for treating or preventing HCV infection.

26. Use of a compound that blocks a process of sphingomyelin biosynthesis in the preparation of a pharmaceutical composition for treating or preventing HCV infection which is used in combination with an interferon.

27. Use of an interferon in the preparation of a pharmaceutical composition for treating or preventing HCV infection which is used in combination with a compound that blocks a process of sphingomyelin biosynthesis.

28. Use of any one of claims 25 to 27, wherein the compound that blocks a process of sphingomyelin biosynthesis is a compound that blocks a process of biosynthesis of sphingomyelin from palmitoyl CoA.

29. Use of any one of claims 25 to 27, wherein the compound that blocks a process of sphingomyelin biosynthesis is:
(a) a compound that inhibits the enzymatic activity of a serine palmitoyltransferase involved in the biosynthesis of 3-ketodihydrosphingosine from palmitoyl CoA; or
(b) a compound that suppresses the expression of a serine palmitoyltransferase.

30. The use of claim 29, wherein the compound that inhibits the enzymatic activity of a serine palmitoyltransferase is myriocin, sphingofungin, a compound represented by formula (I), or a pharmaceutically acceptable salt thereof: 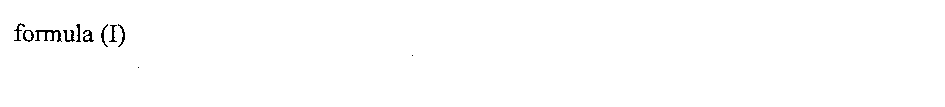 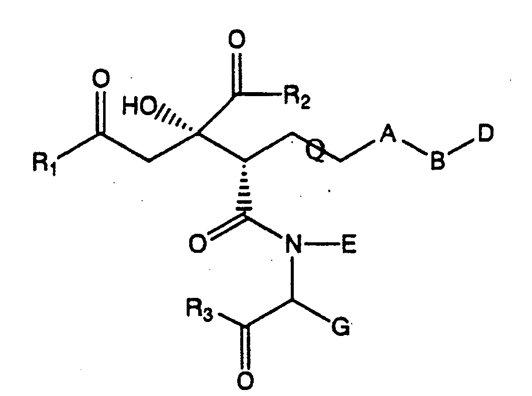 wherein
A represents -(CH₂)ₙ-, wherein n represents an integer from 0 to 10;
B represents -CH₂-, -(C=O)-, -CH(OH)-, -CH(NH₂)-, or -C(=NOR)-, wherein R represents a hydrogen atom, linear or branched alkyl group having 1 to 8 carbon atoms, which is optionally substituted by an amino group which is optionally mono- or di-substituted by a linear or branched alkyl group having 1 to 4 carbon atoms);
D represents -(CH₂)ₘ-R', wherein m represents an integer from 0 to 10, and R' represents a hydrogen atom, linear or branched alkyl group, linear or branched alkynyl group, linear or branched alkenyl group, cycloalkyl group, cycloalkenyl group, optionally substituted heterocyclic group, optionally substituted aryl group, optionally substituted heteroaryl group, -OX group (wherein X represents a hydrogen atom, linear or branched alkyl group, linear or branched alkynyl group, linear or branched alkenyl group, cycloalkyl group, or optionally substituted aryl group), or halogen atom;
E represents a hydrogen atom, or linear or branched alkyl group;
G represents -(CH₂)ₚ-J, wherein p represents an integer from 0 to 4; and J represents a hydrogen, OH group, SH group, methylthio group, carboxyl group, carbamoyl group, amino group, guanidino group, linear or branched alkyl group, cycloalkyl group, linear or branched alkynyl group, linear or branched alkenyl group, optionally substituted aryl group, optionally substituted heterocyclic group, or optionally substituted heteroaryl group;
bond Q represents a single bond or a double bond; and
R¹, R², and R³ are the same or different and each represent a hydroxyl group, amino group (which is optionally mono- or di-substituted by a linear or branched alkyl group having 1 to 4 carbon atoms), -OL, linear or branched alkyl group, linear or branched alkenyl group, or linear or branched alkynyl group, wherein L represents a linear or branched alkyl group, linear or branched alkenyl group, or linear or branched alkynyl group.

31. The use of claim 29, wherein the compound that inhibits the enzymatic activity of a serine palmitoyltransferase is a compound represented by any one of formulae (II) to (XII), or a pharmaceutically acceptable salt thereof:

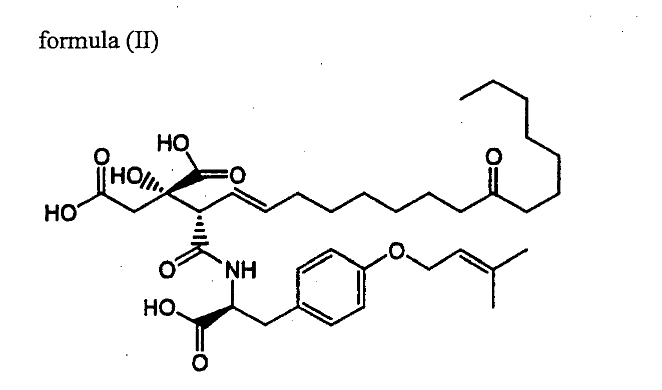

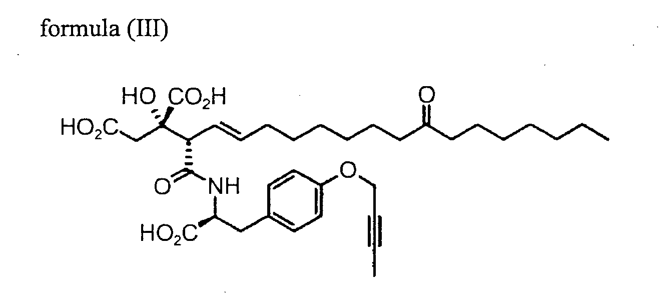

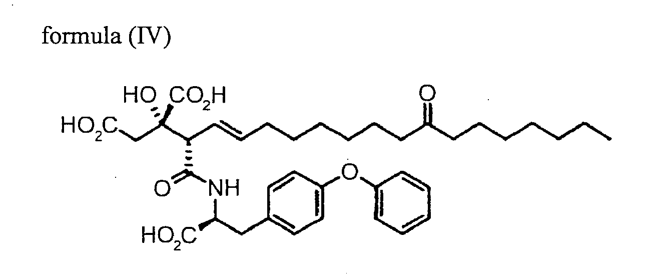

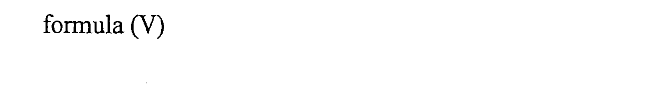

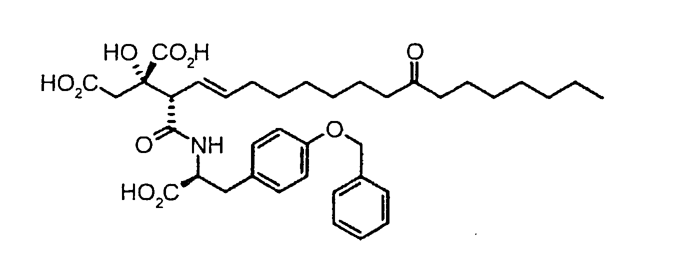

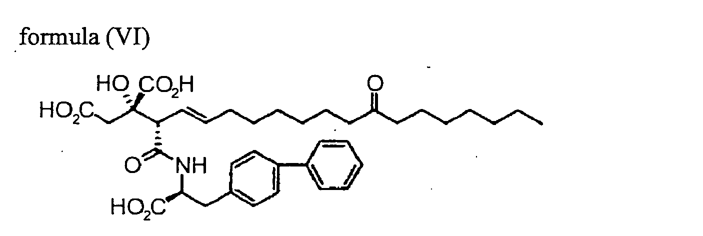

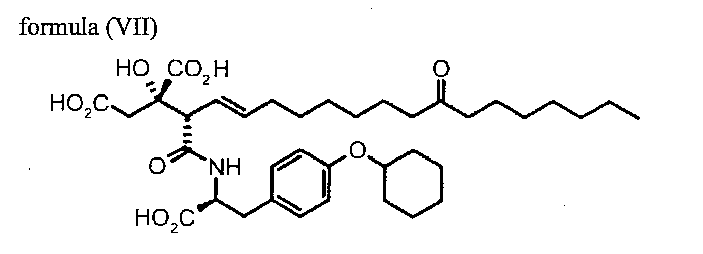

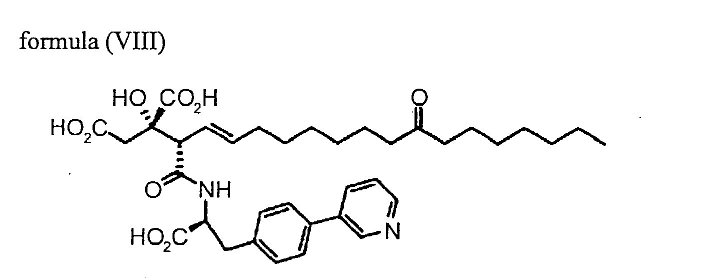

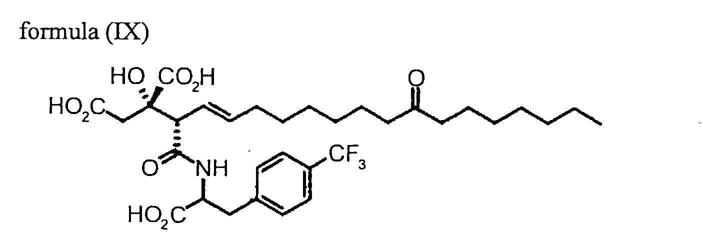

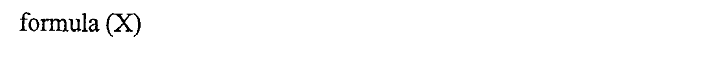

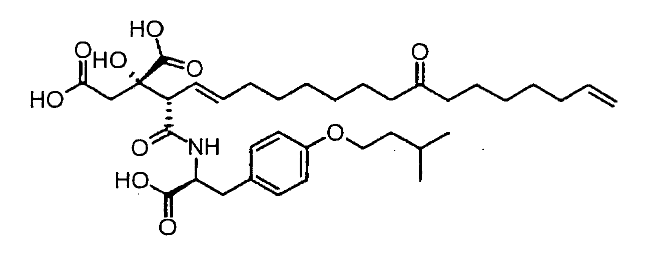

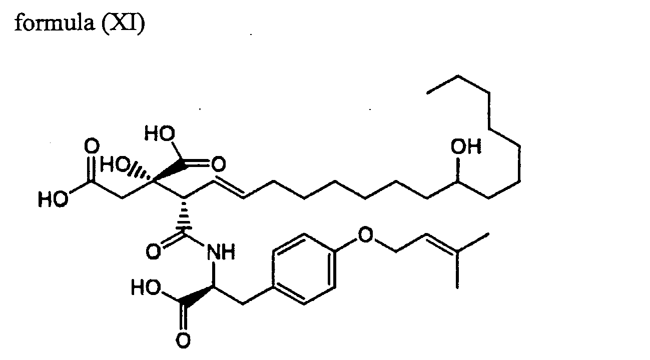

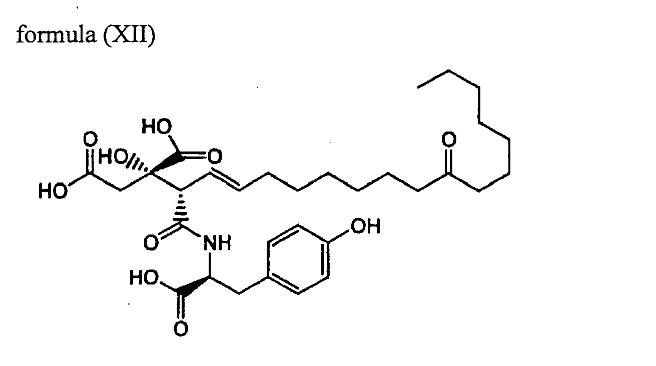

32. The use of claim 29, wherein the compound that suppresses the expression of a serine palmitoyltransferase is:
(a) an RNA complementary to a transcript of DNA encoding a serine palmitoyltransferase; or
(b) an RNA having ribozyme activity to specifically cleave a transcript of DNA encoding a serine palmitoyltransferase.

33. The use of any one of claims 25 to 27, wherein the interferon is a pegylated interferon.

34. The use of any one of claims 25 to 27, wherein the HCV infection is type C hepatitis, cirrhosis, hepatic fibrosis, or hepatic cancer.
